# EUROPEAN PATENT APPLICATION

(11) **EP 3 620 449 A1**
(43) Date of publication of application: **11.03.2020**
(21) Application number: 18876048.2
(22) Date of filing: 14.09.2018
(51) Int. Cl.: C07C 211/61, H01L 51/00, H01L 51/50, C09K 11/06

(54) **NOVEL COMPOUND AND ORGANIC LIGHT-EMITTING ELEMENT USING SAME**

(30) Priority: 10.11.2017 KR 20170149680; 13.09.2018 KR 20180109532
(71) Applicant: LG Chem, Ltd., Seoul 07336 (KR)
(72) Inventor: HA, Jae Seung, Daejeon 34122 (KR); LEE, Sung Jae, Daejeon 34122 (KR); MUN, Hyeon Jin, Daejeon 34122 (KR)
(74) Representative: Hoffmann Eitle
(86) International application number: PCT/KR2018/010867
(87) International publication number: WO 2019/093649

(57) **Abstract**

The present invention relates to a novel compound and an organic light emitting device using the same.

## Description

### [Technical Field]

This application claims priority to and the benefits of Korean Patent Application No. 10-2017-0149680 filed on November 10, 2017, the disclosure of which is incorporated herein by reference in its entirety.

The present invention relates to a novel compound and an organic light emitting device comprising the same.

### [Background Art]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently have a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and the electrons meet each other, an exciton is formed, and light is emitted when the exciton falls to a ground state again.

There is a continuing need for developing new materials as the organic materials used in the aforementioned organic light emitting devices.

### [Prior Art Literature]

### [Patent Literature]

(Patent Literature 0001) Korean Patent Laid-open Publication No. 10-2000-0051826

### [DETAILED DESCRIPTION OF THE INVENTION]

### [Technical Problem]

The present invention relates to a novel compound and an organic light emitting device comprising the same.

### [Technical Solution]

The present invention provides a compound represented by the following Chemical Formula 1: 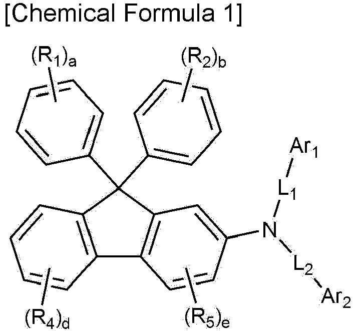 in Chemical Formula 1,
R₁, R₂, R₄ and R₅ are each independently hydrogen, deuterium, halogen, cyano, a substituted or unsubstituted C₁₋₆₀ alkyl, a substituted or unsubstituted C₁₋₆₀ alkoxy, a substituted or unsubstituted C₁₋₆₀ thioalkyl, a substituted or unsubstituted C₃₋₆₀ cycloalkyl, a substituted or unsubstituted C₆₋₆₀ aryl, or a tri(C₁₋₆₀ alkyl)silyl,
a, b and d are each independently an integer of 0 to 4,
e is an integer of 0 to 3,
L₁ and L₂ are each independently a single bond; a substituted or unsubstituted C₆₋₆₀ arylene; or a substituted or unsubstituted C₂₋₆₀ heteroarylene containing at least one hetero atom selected from the group consisting of N, O, and S, and
Ar₁ is represented by the following Chemical Formula 2, 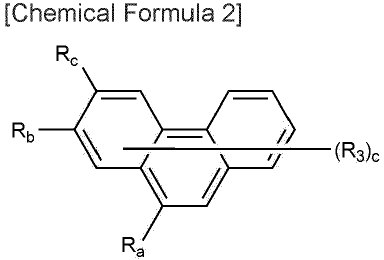 in Chemical Formula 2,
   one of Rₐ, R_{b} and R_{c} is bonded to L₁ and the rest are hydrogen,
   R₃ is hydrogen, deuterium, halogen, cyano, a substituted or unsubstituted C₁₋₆₀ alkyl, a substituted or unsubstituted C₁₋₆₀ alkoxy, a substituted or unsubstituted C₁₋₆₀ thioalkyl, a substituted or unsubstituted C₃₋₆₀ cycloalkyl, a substituted or unsubstituted C₆₋₆₀ aryl, or a tri(C₁₋₆₀ alkyl)silyl,
   c is an integer of 0 to 7, and
   Ar₂ is any one aryl selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b; or any one heteroaryl selected from the group consisting of dibenzofuranyl and dibenzothiophenyl, wherein the Ar₂ is unsubstituted or substituted with one to five deuterium atoms, 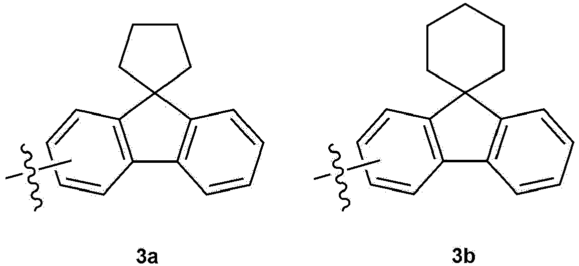 provided that when R_{b} is bonded to L₁, Ar₂ is the aryl.

The present invention also provides an organic light emitting device comprising a first electrode; a second electrode provided at a side opposite to the first electrode; and at least one layer of the organic material layers provided between the first electrode and the second electrode, wherein the at least one layer of the organic material layers includes a compound represented by Chemical Formula 1.

### [ADVANTAGEOUS EFFECTS]

The compound represented by Chemical Formula 1 as described above can be used as a material of the organic material layer of the light emitting device, and enables improvement of the efficiency, low driving voltage and/or improvement of the lifetime characteristic when applied to the organic light emitting device. In particular, the compound represented by Chemical Formula 1 can be used as hole injection, hole transport, hole injection and transport, hole adjustment, light emitting, electron transport, or electron injection materials.

### [BRIEF DESCRIPTION OF DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a hole adjustment layer 7, a light emitting layer 3, an electron transport layer 8, and a cathode 4.

### [DETAILED DESCRIPTION OF THE EMBODIMENTS]

Hereinafter, the present invention will be described in more detail to help understanding of the present invention.

The present invention provides a compound represented by Chemical Formula 1.

In the present specification, the notation 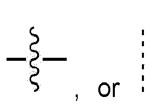 , or mean a bond connected to another substituent group.

As used herein, the term "substituted or unsubstituted" means being unsubstituted or substituted by one or more substituents selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing at least one of N, O, and S atoms, or being unsubstituted or substituted by a substituent to which two or more substituents are linked among the exemplified substituents. For example, "the substituent to which two or more substituents are linked" may be a biphenyl group. That is, the biphenyl group may be an aryl group, or may be interpreted as a substituent to which two phenyl groups are linked.

In the present specification, the number of carbon atoms in a carbonyl group is not particularly limited, but is preferably 1 to 40. Specifically, the carbonyl group may be compounds having the following structures, but is not limited thereto.

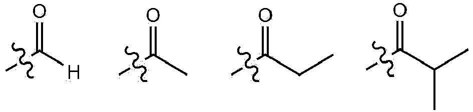

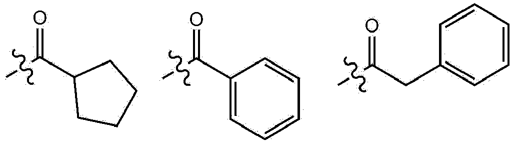

In the present specification, the ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be compounds having the following structures, but is not limited thereto.

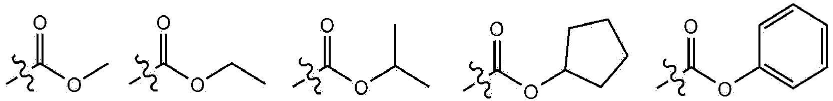

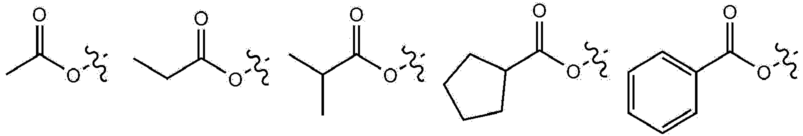

In the present specification, the number of carbon atoms in an imide group is not particularly limited, but is preferably 1 to 25. Specifically, the imide group may be compounds having the following structures, but is not limited thereto.

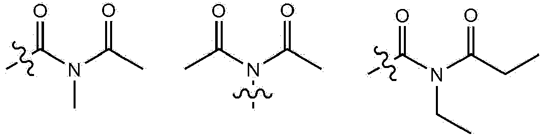

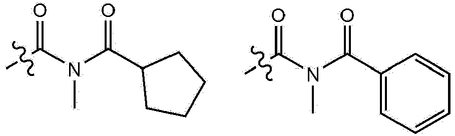

In the present specification, the silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present specification, the boron group specifically includes a trimethylboron group, a triethylboron group, a t-butyldimethylboron group, a triphenylboron group, a phenylboron group, and the like, but is not limited thereto.

In the present specification, examples of a halogen group include fluorine, chlorine, bromine, or iodine.

In the present specification, the alkyl group may be a straight chain or a branched chain, and the number of carbon atoms thereof is not particularly limited, but is preferably 1 to 40. According to one embodiment, the alkyl group has 1 to 20 carbon atoms. According to another embodiment, the alkyl group has 1 to 10 carbon atoms. According to still another embodiment, the alkyl group has 1 to 6 carbon atoms. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present specification, the alkenyl group may be a straight chain or a branched chain, and the number of carbon atoms thereof is not particularly limited, but is preferably 2 to 40. According to one embodiment, the alkenyl group has 2 to 20 carbon atoms. According to another embodiment, the alkenyl group has 2 to 10 carbon atoms. According to still another embodiment, the alkenyl group has 2 to 6 carbon atoms. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, the cycloalkyl group is not particularly limited, but the number of carbon atoms thereof is preferably 3 to 60. According to one embodiment, the cycloalkyl group has 3 to 30 carbon atoms. According to another embodiment, the cycloalkyl group has 3 to 20 carbon atoms. According to another embodiment, the cycloalkyl group has 3 to 6 carbon atoms. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, the aryl group is not particularly limited, but preferably has 6 to 60 carbon atoms, and may be a monocyclic aryl group or a polycyclic aryl group. According to one embodiment, the aryl group has 6 to 30 carbon atoms. According to one embodiment, the aryl group has 6 to 20 carbon atoms. The aryl group may be a phenyl group, a biphenyl group, a terphenyl group or the like as the monocyclic aryl group, but is not limited thereto. Examples of the polycyclic aryl group include a naphthyl group, an anthracenyl group, a phenanthryl group, a pyrenyl group, a perylenyl group, a chrycenyl group, a fluorenyl group or the like, but is not limited thereto.

In the present specification, a fluorenyl group may be substituted, and two substituents may be linked with each other to form a spiro structure. In the case where the fluorenyl group is substituted, 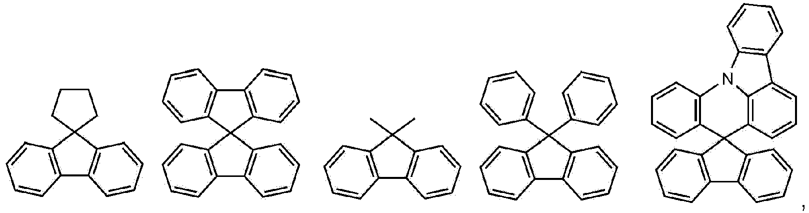 and the like can be formed. However, the structure is not limited thereto.

In the present specification, the heterocyclic group is a heterocyclic group containing at least one of O, N, Si and S as a heteroatom, and the number of carbon atoms thereof is not particularly limited, but is preferably 2 to 60. Examples of the heterocyclic group include a thiophene group, a furan group, a pyrrole group, an imidazole group, a thiazole group, an oxazole group, an oxadiazole group, a triazole group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present specification, the aryl group in the aralkyl group, the aralkenyl group, the alkylaryl group, and the arylamine group is the same as the aforementioned examples of the aryl group. In the present specification, the alkyl group in the aralkyl group, the alkylaryl group and the alkylamine group is the same as the aforementioned examples of the alkyl group. In the present specification, the heteroaryl in the heteroarylamine can be applied to the aforementioned description of the heterocyclic group. In the present specification, the alkenyl group in the aralkenyl group is the same as the aforementioned examples of the alkenyl group. In the present specification, the aforementioned description of the aryl group may be applied except that the arylene is a divalent group. In the present specification, the aforementioned description of the heterocyclic group can be applied except that the heteroarylene is a divalent group. In the present specification, the aforementioned description of the aryl group or cycloalkyl group can be applied except that the hydrocarbon ring is not a monovalent group but formed by combining two substituents. In the present specification, the aforementioned description of the heterocyclic group can be applied, except that the heterocycle is not a monovalent group but formed by combining two substituents.

In Chemical Formula 1, preferably, R₁, R₂, and R₄ are hydrogen. Further, preferably, a, b, and d are 0.

Preferably, R₅ is hydrogen or deuterium. Preferably, e is 3 and R₅ is deuterium.

Preferably, L₁ is a single bond; a phenylene which is unsubstituted or substituted with one to four deuterium atoms; or a biphenylylene which is unsubstituted or substituted with one to eight deuterium atoms. Preferably, L₂ is a single bond; a phenylene which is unsubstituted or substituted with one to four deuterium atoms; or a biphenylylene which is unsubstituted or substituted with one to eight deuterium atoms. More preferably, L₁ and L₂ are each independently a single bond; or one of the following:

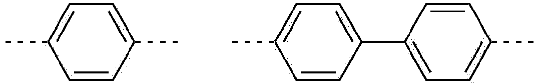

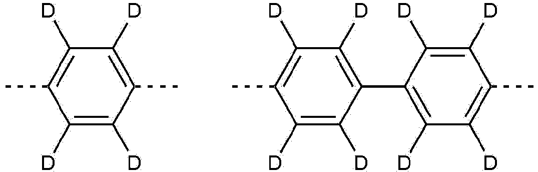

Preferably, R₃ is hydrogen. Further, preferably, c is 0.

Preferably, the compound represented by Chemical Formula 1 is represented by the following Chemical Formula 1-1 or 1-2.

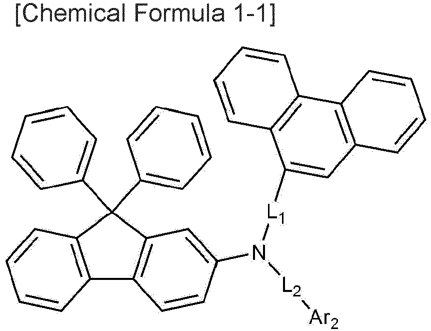

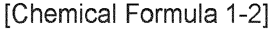

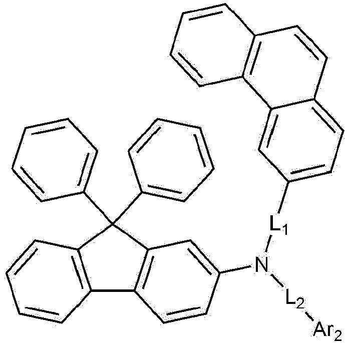

in Chemical Formulae 1-1 and 1-2,
L₁ and L₂ are each independently a single bond; or phenylene,
Ar₂ is any one aryl group selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b; or any one heteroaryl selected from the group consisting of dibenzofuranyl and dibenzothiophenyl, wherein the Ar₂ is unsubstituted or substituted with one to five deuterium atoms:

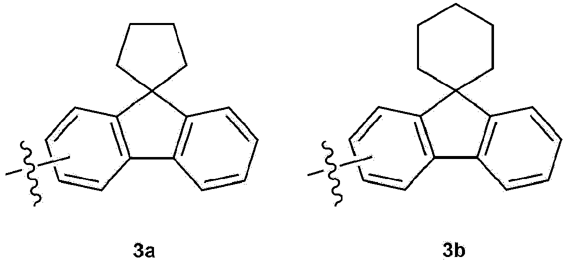

Preferably, the compound represented by Chemical Formula 1 is represented by the following Chemical Formula 1-3:

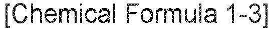

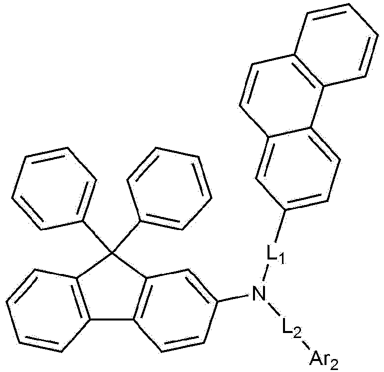

in Chemical Formula 1-3,
L₁ and L₂ are each independently a single bond; or phenylene,
Ar₂ is any one aryl group selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b, wherein the Ar₂ is unsubstituted or substituted with one to five deuterium atoms:

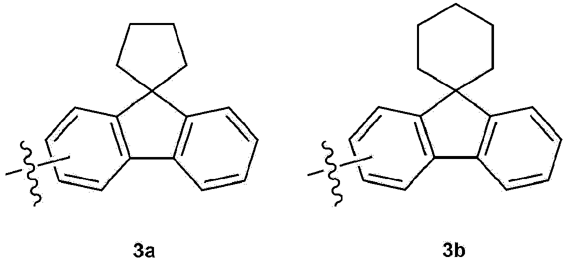

Representative examples of the compound represented by Chemical Formula 1 are as follows:

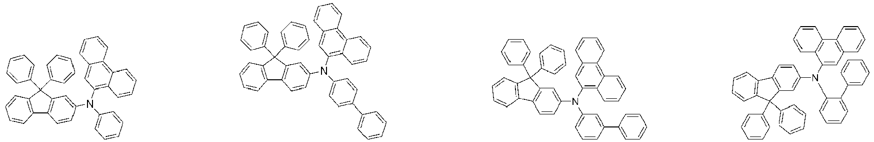

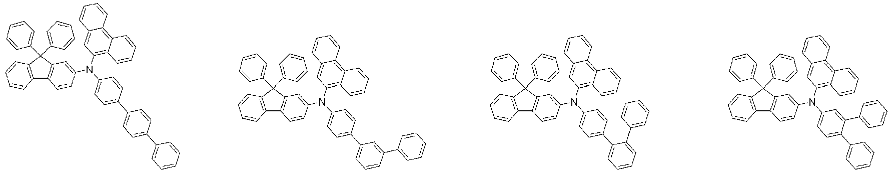

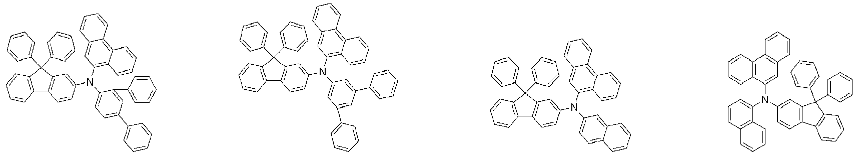

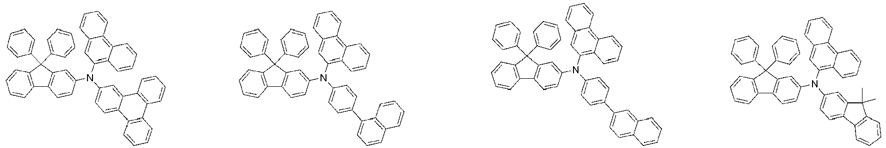

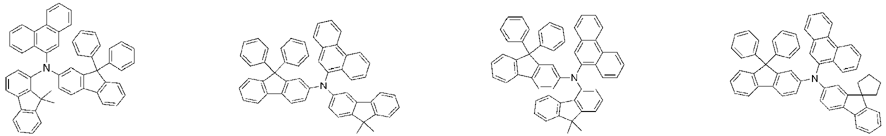

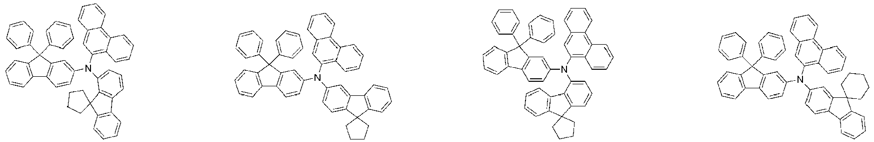

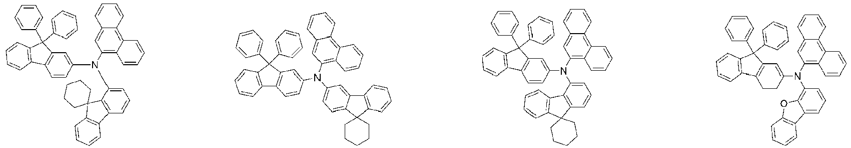

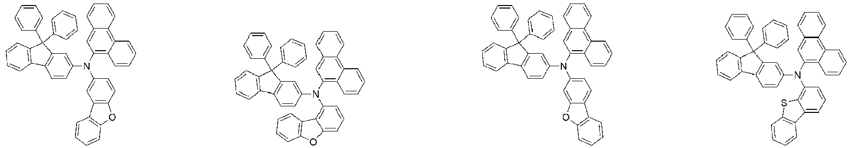

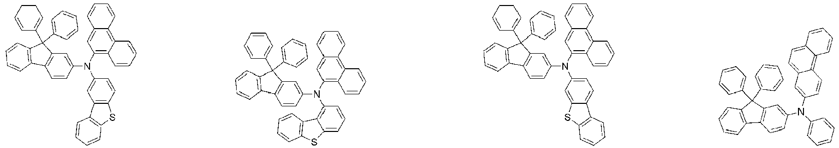

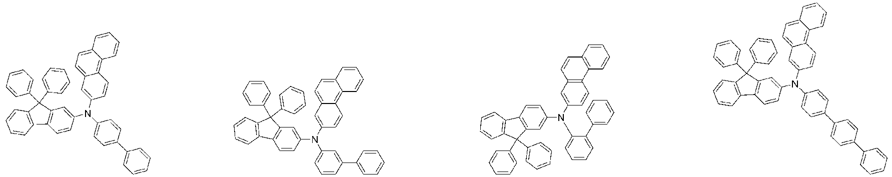

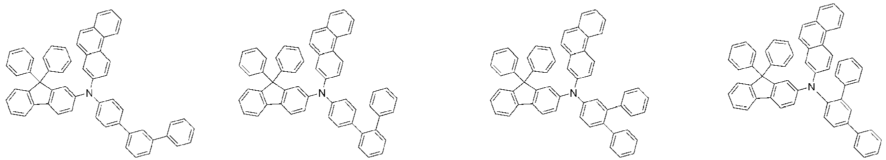

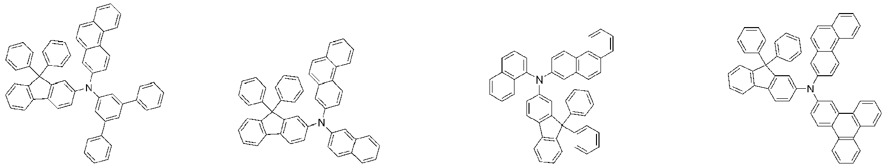

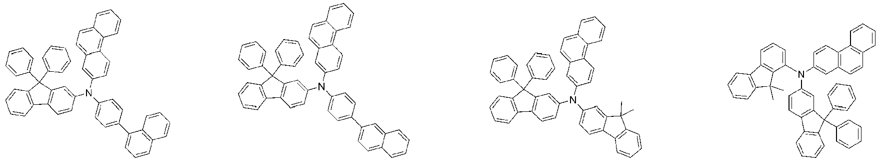

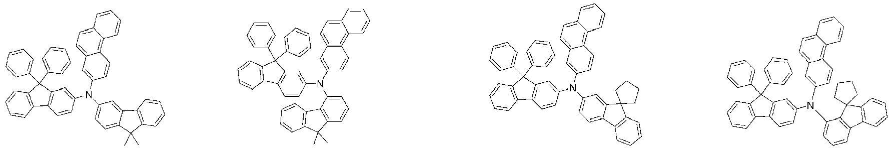

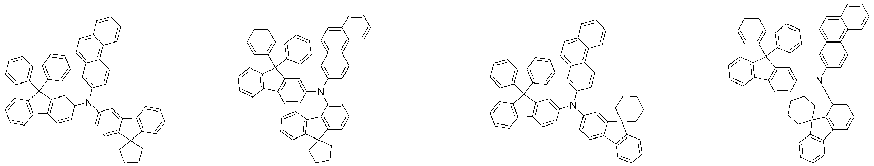

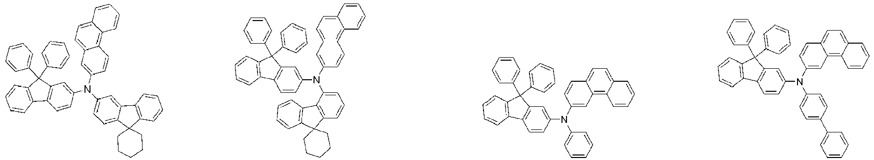

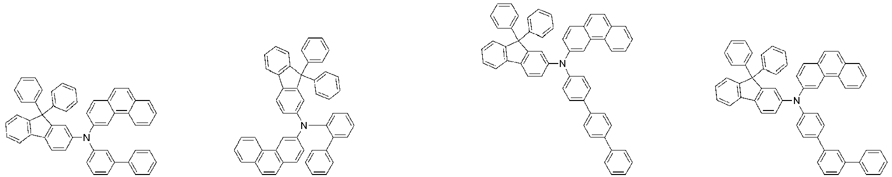

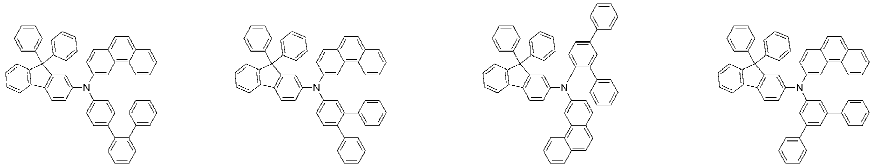

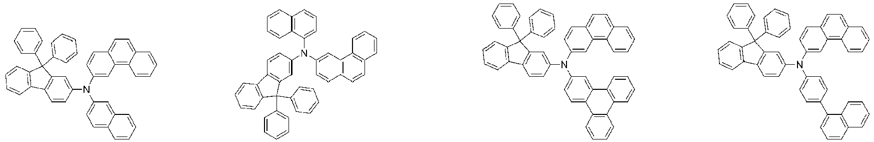

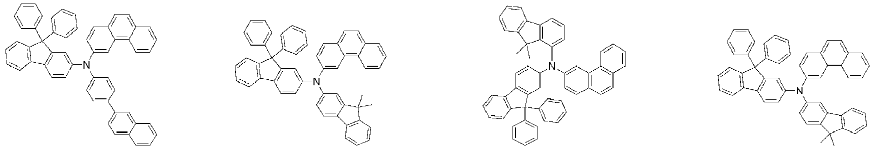

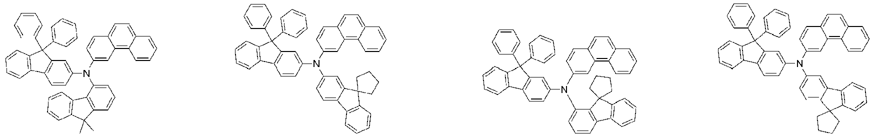

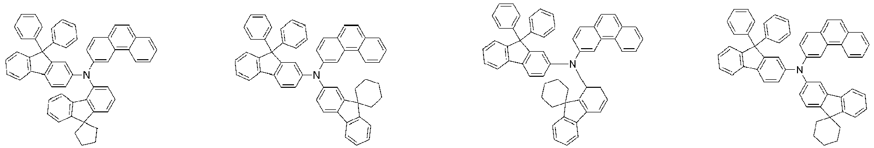

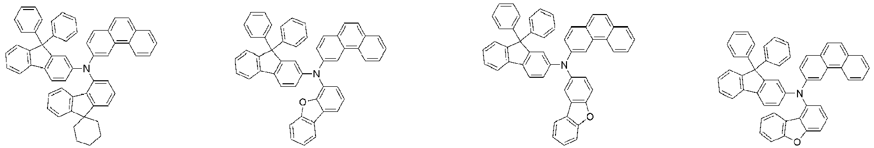

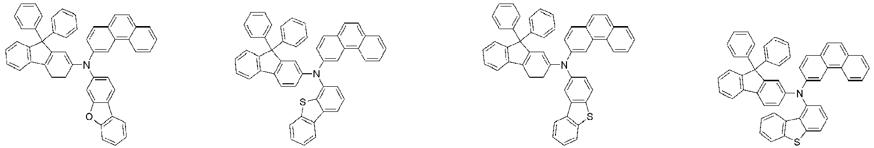

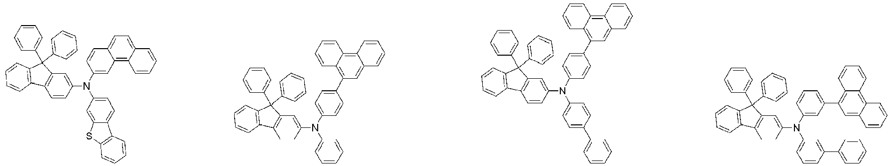

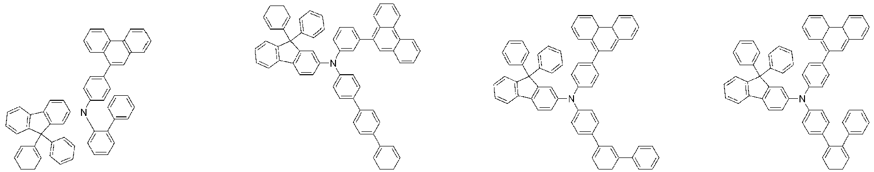

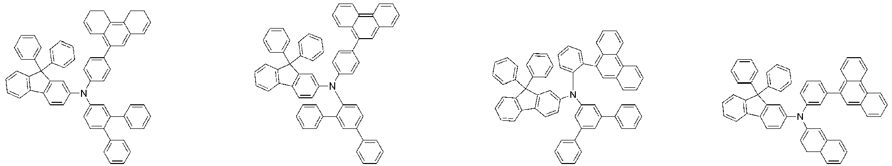

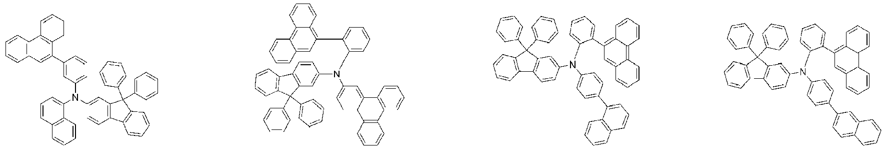

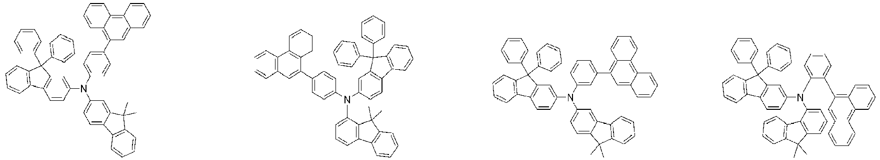

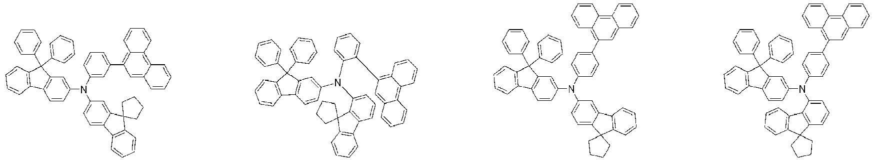

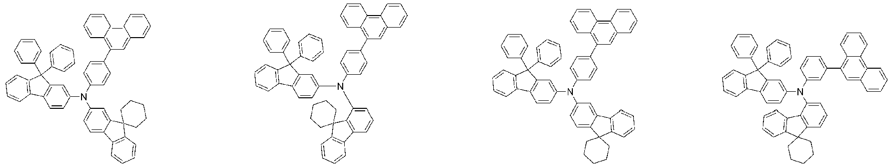

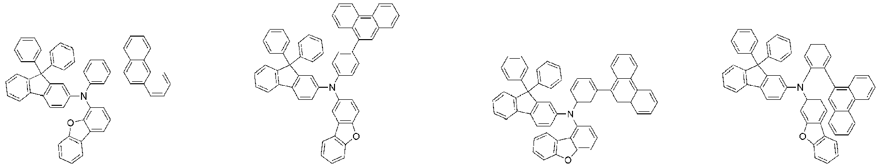

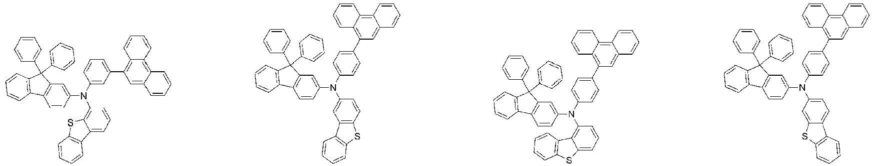

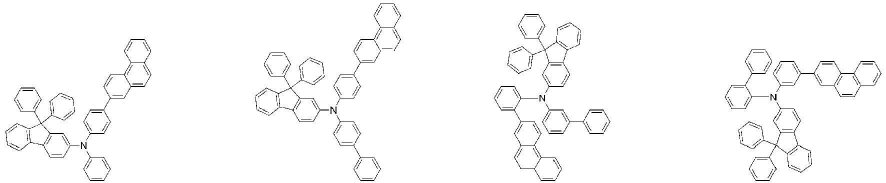

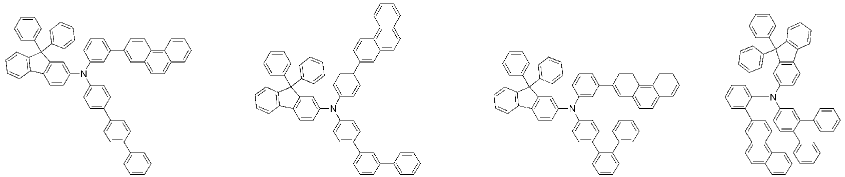

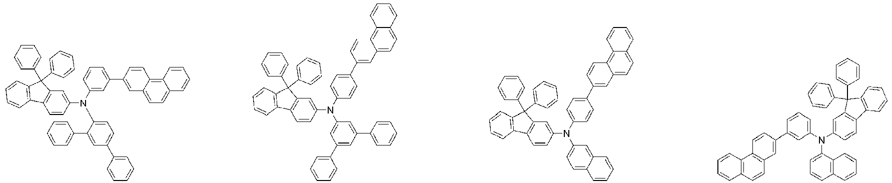

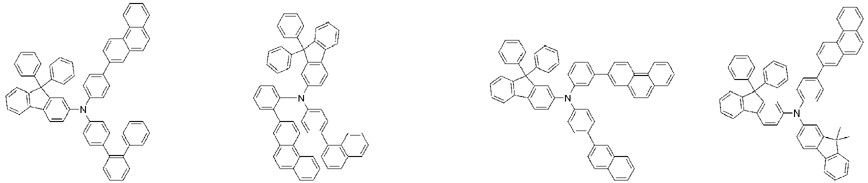

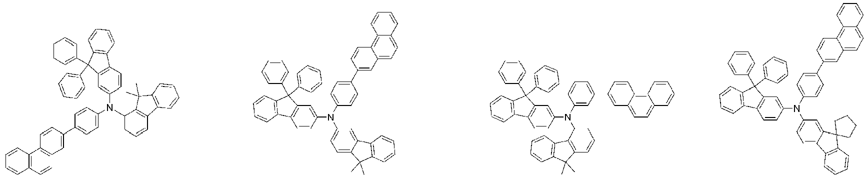

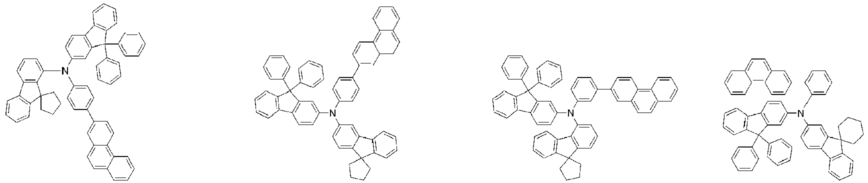

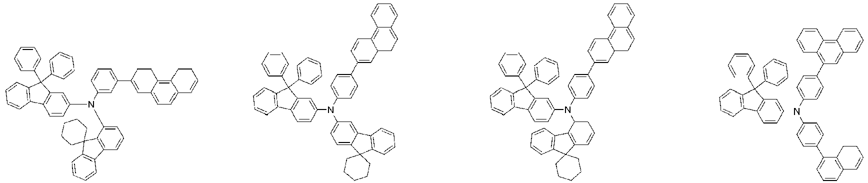

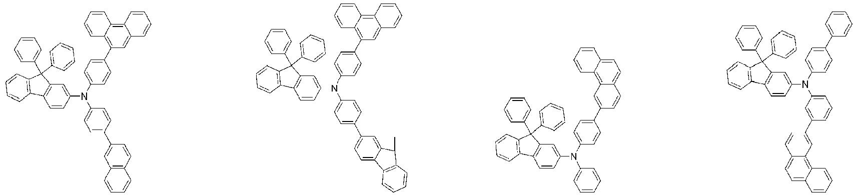

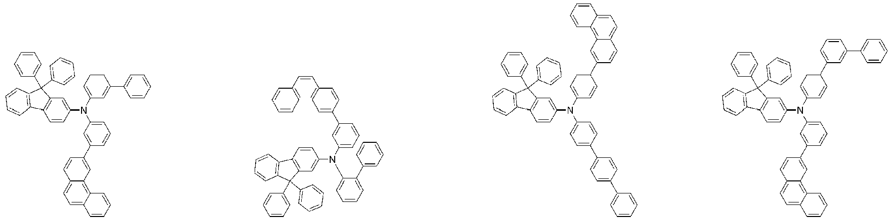

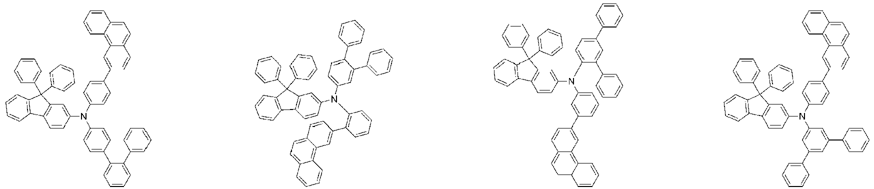

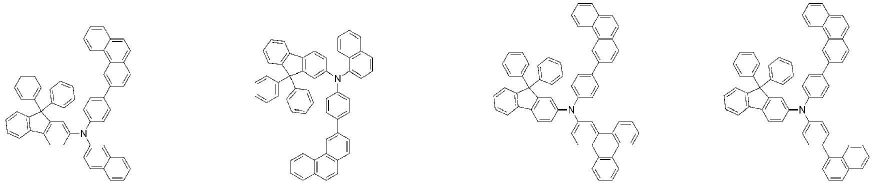

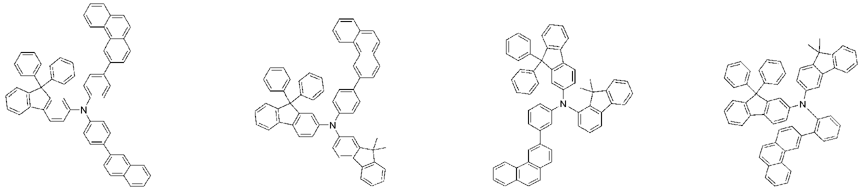

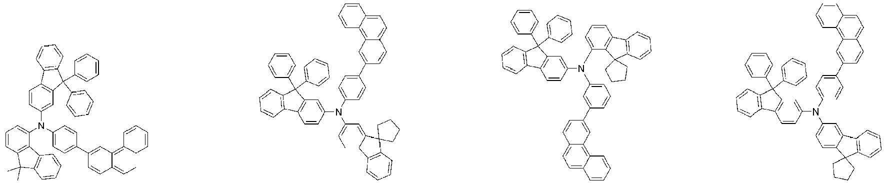

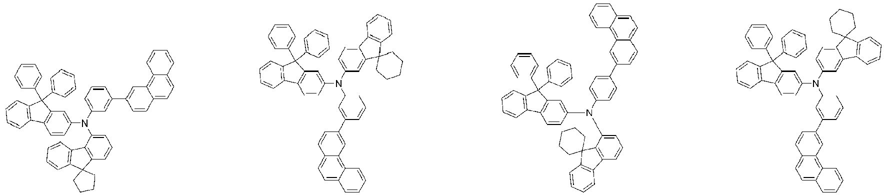

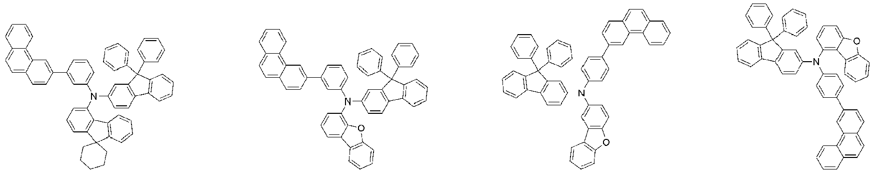

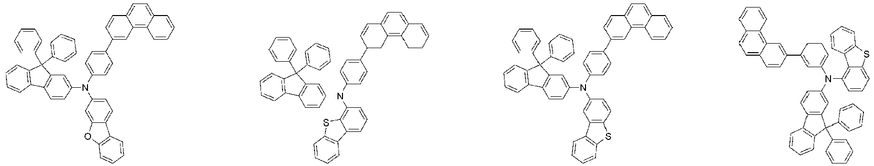

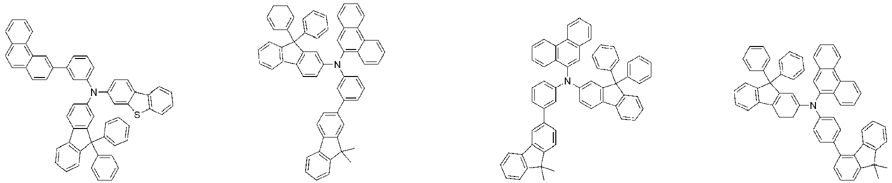

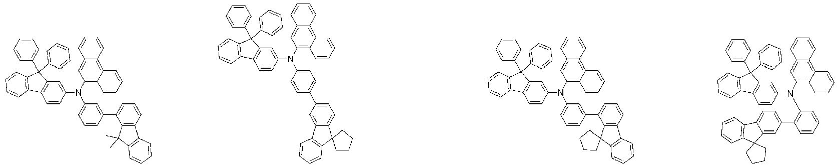

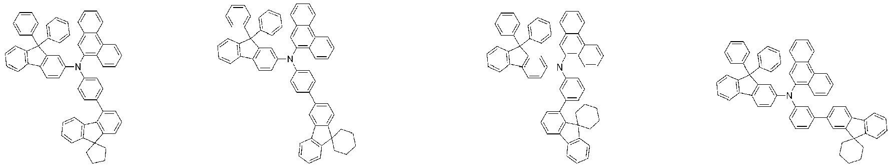

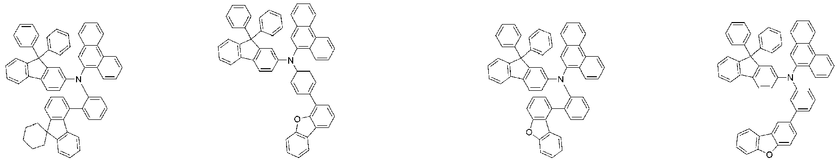

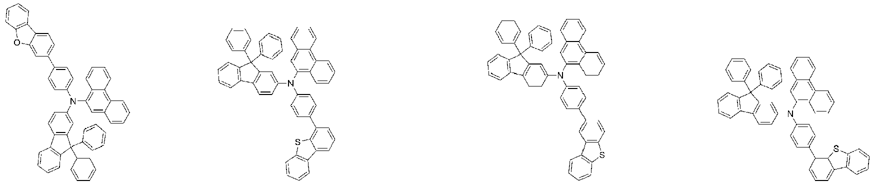

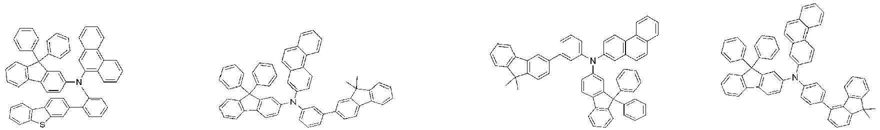

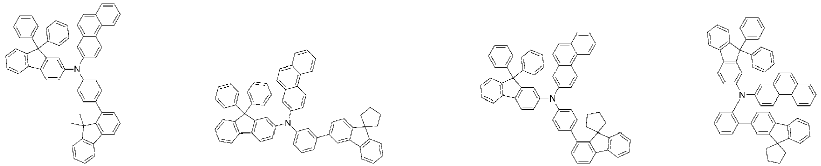

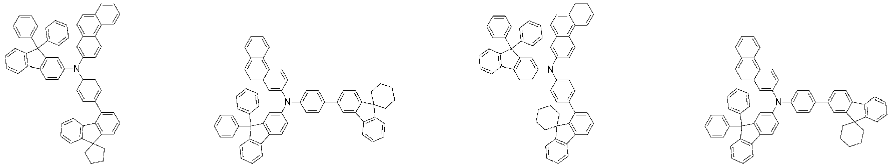

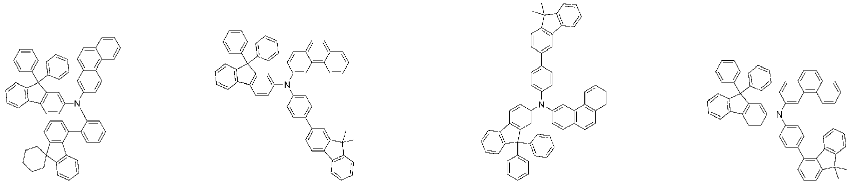

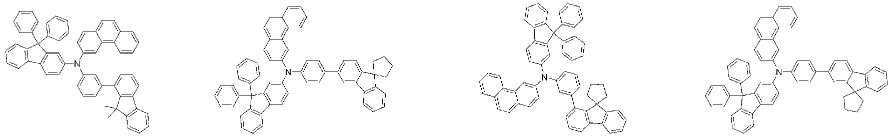

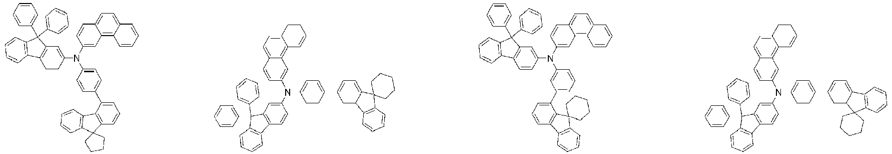

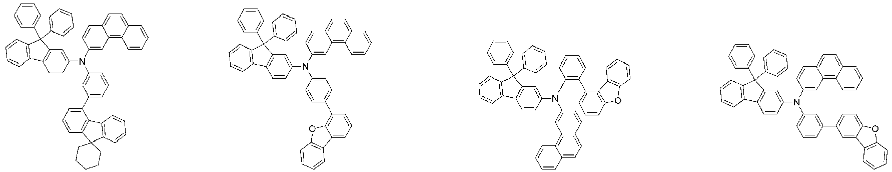

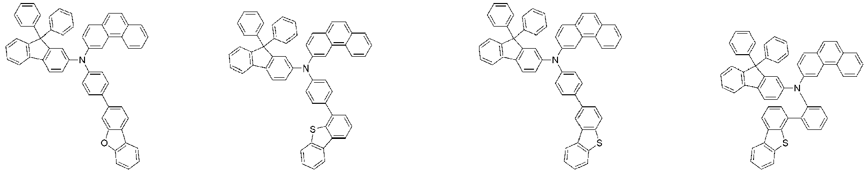

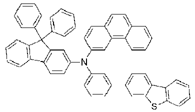

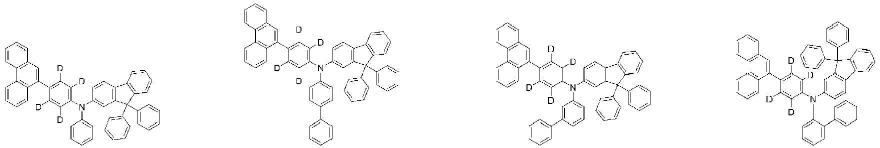

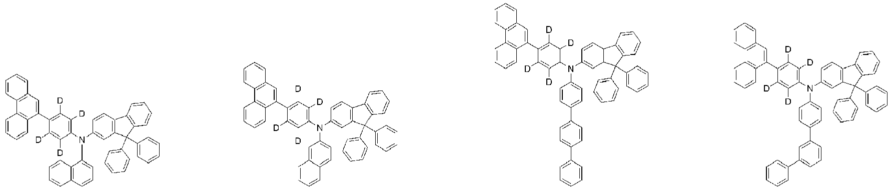

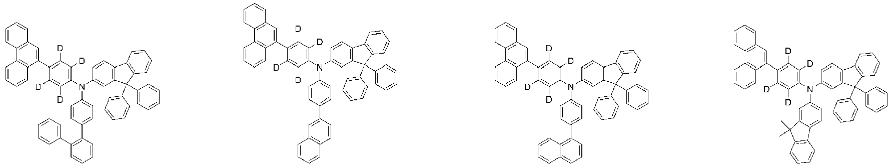

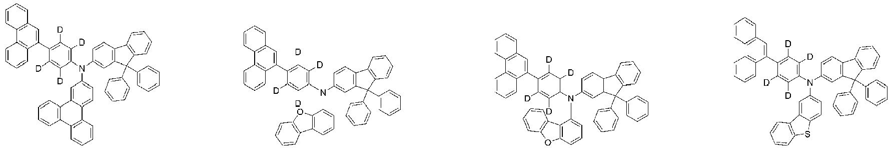

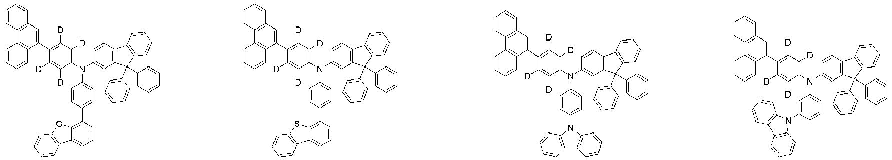

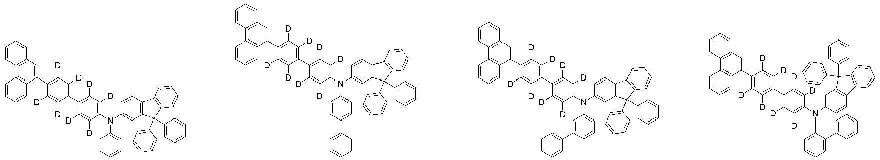

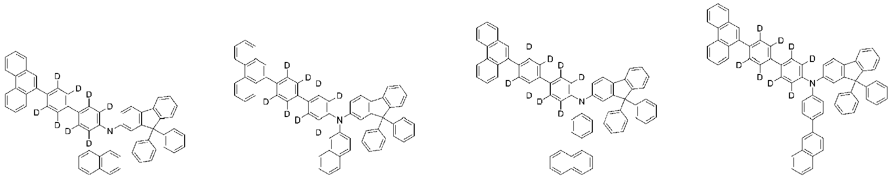

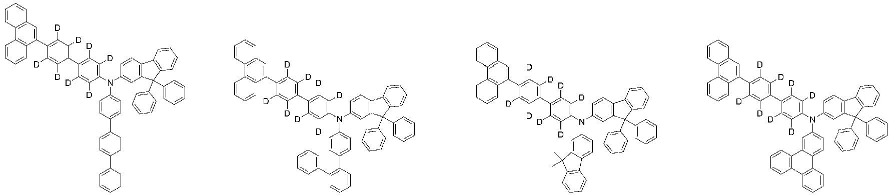

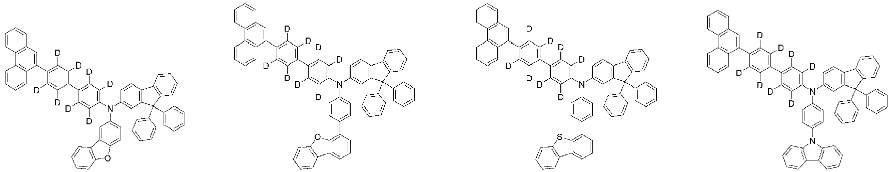

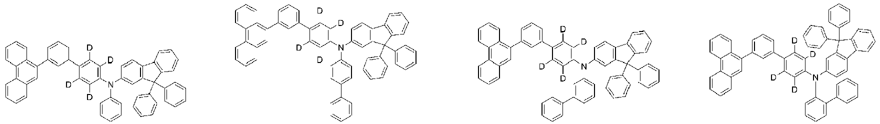

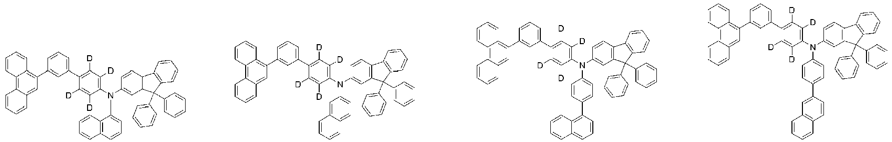

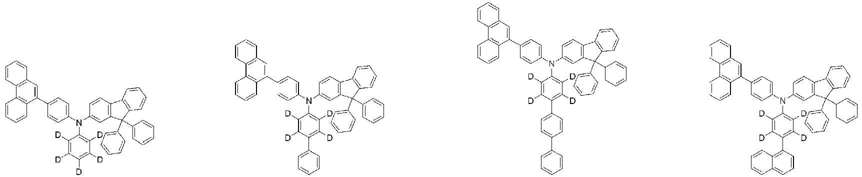

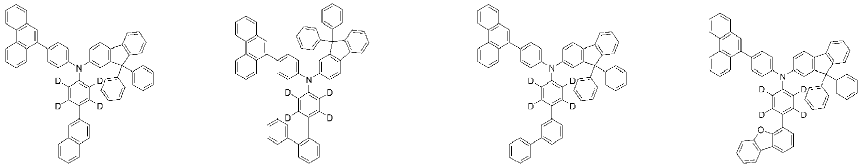

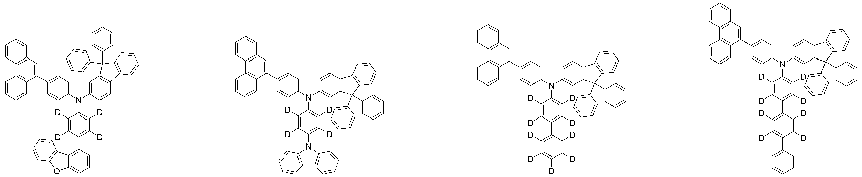

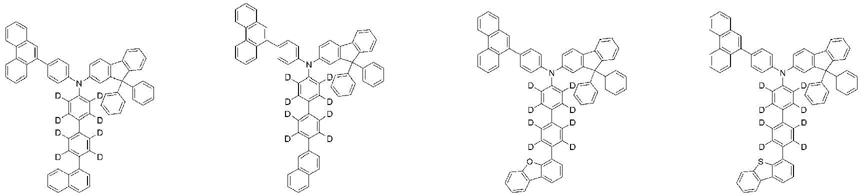

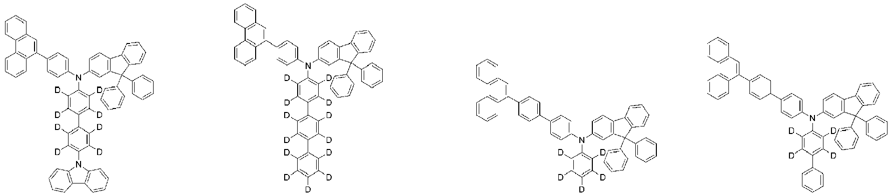

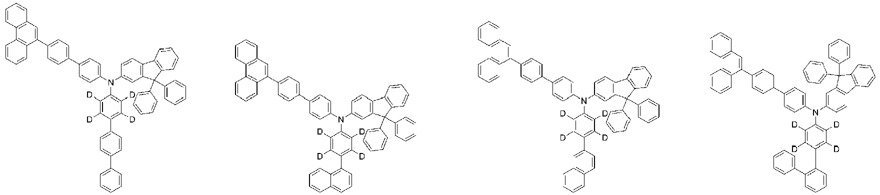

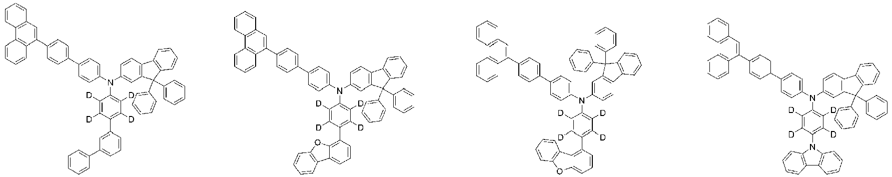

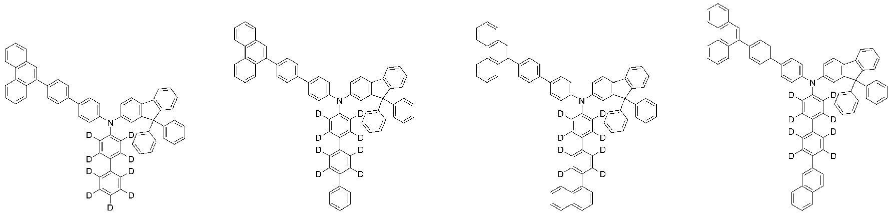

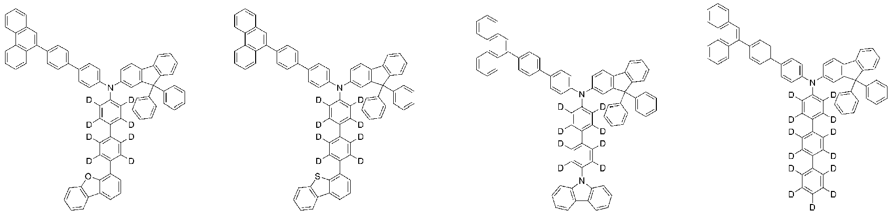

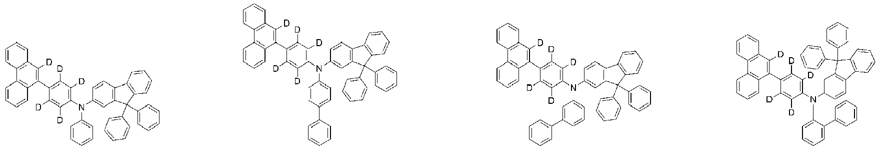

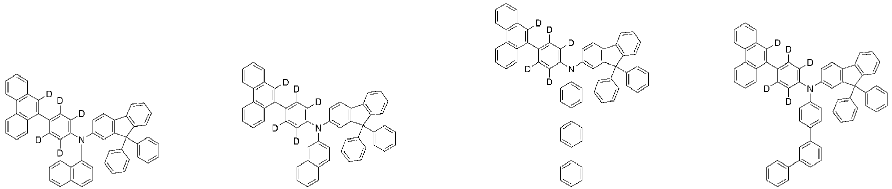

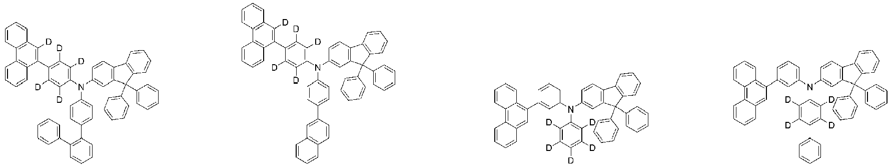

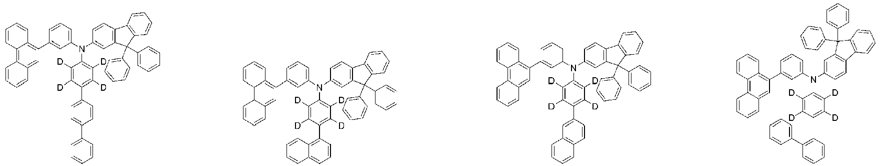

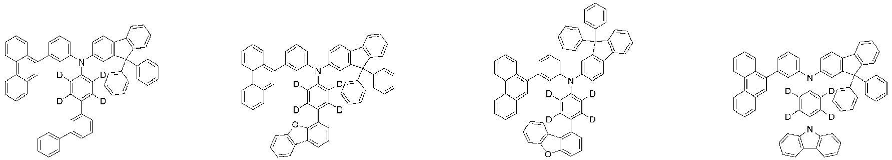

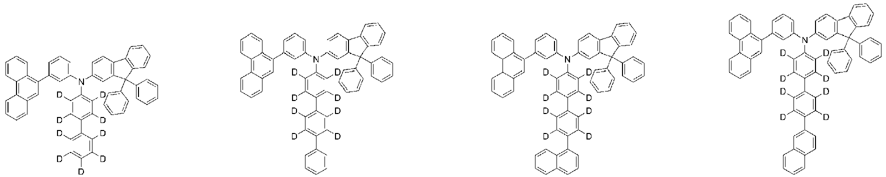

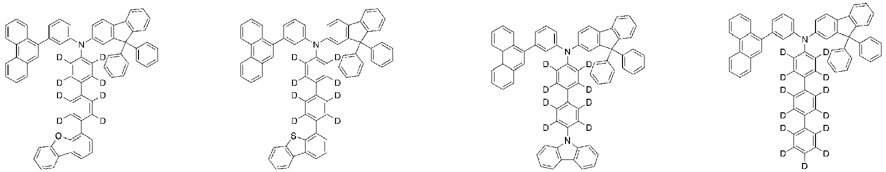

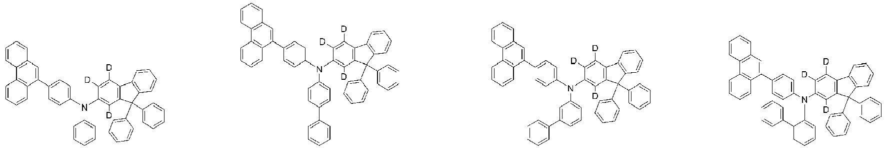

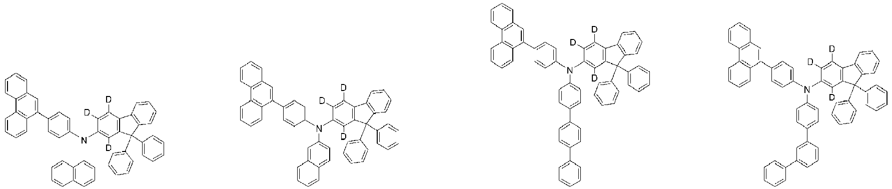

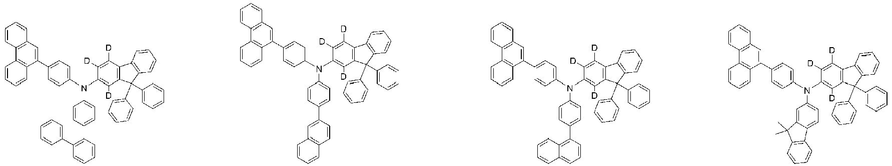

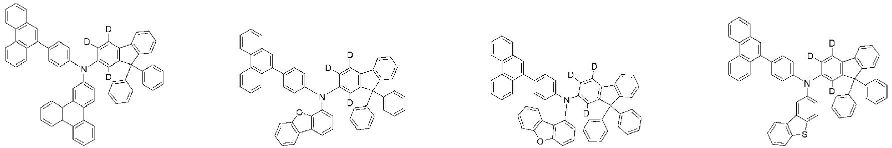

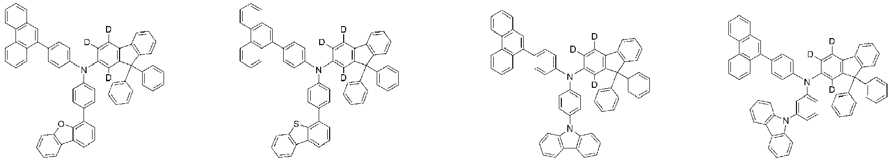

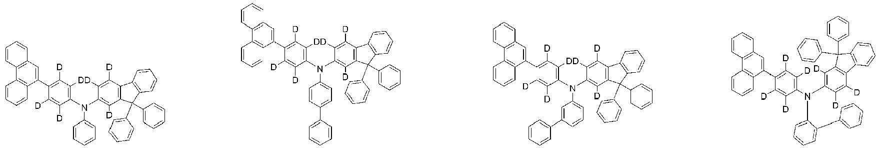

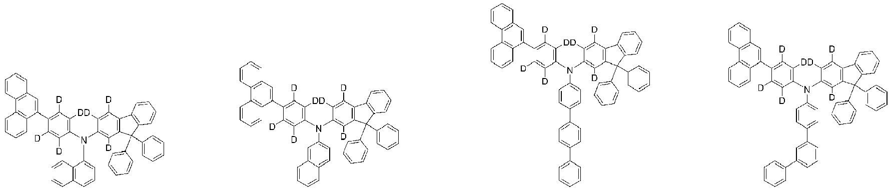

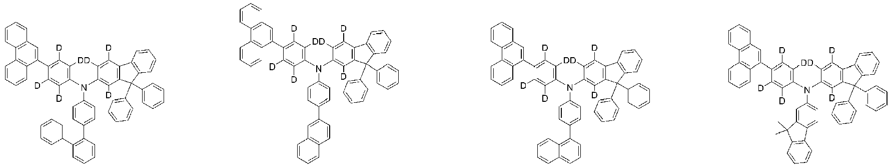

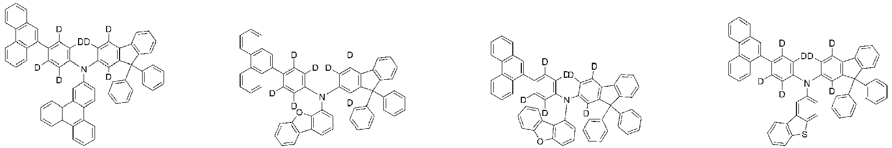

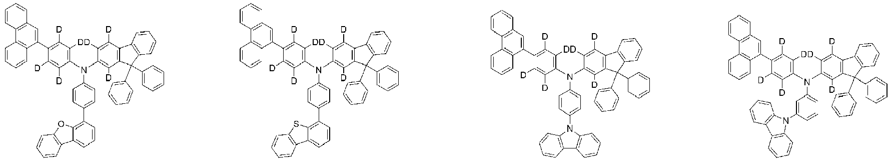

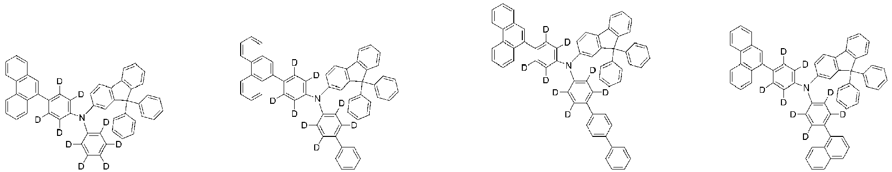

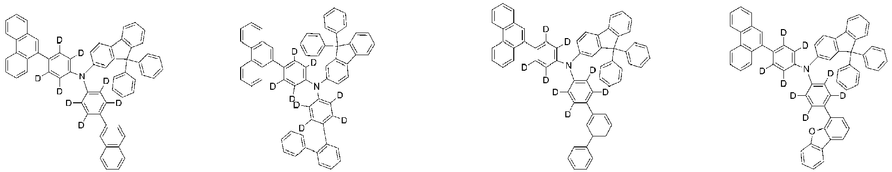

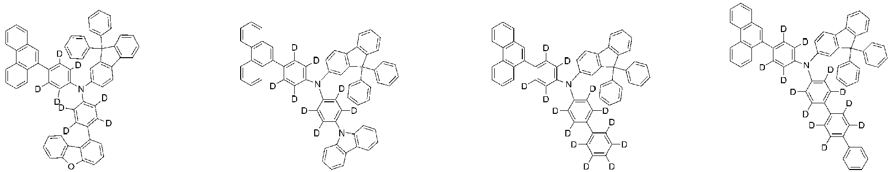

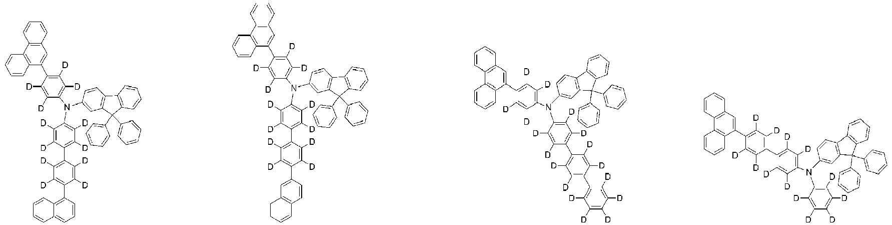

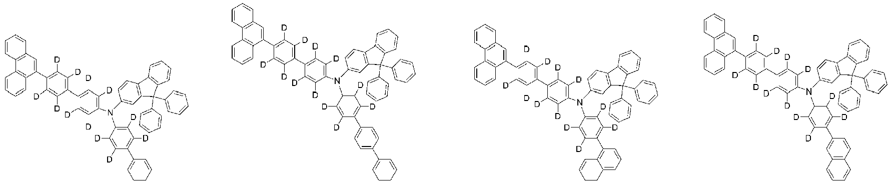

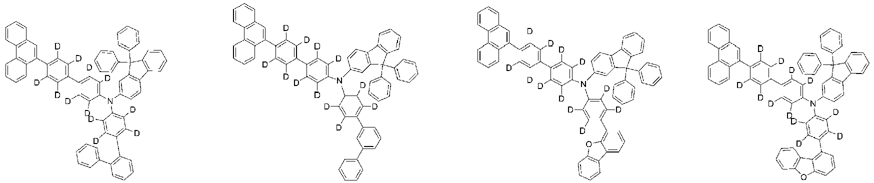

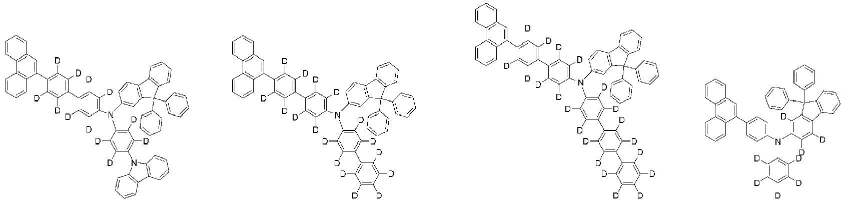

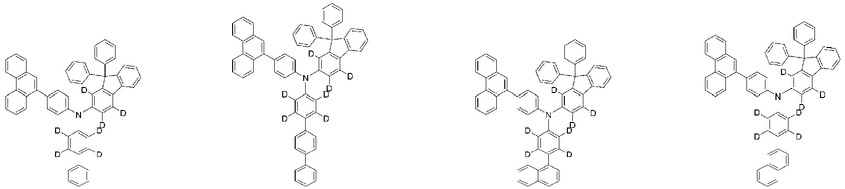

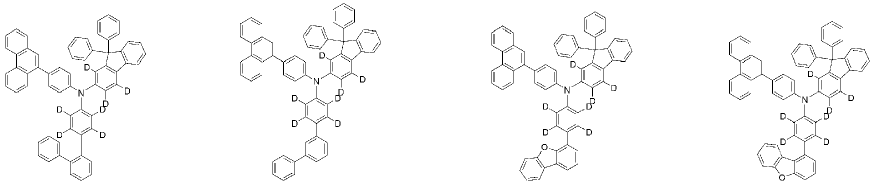

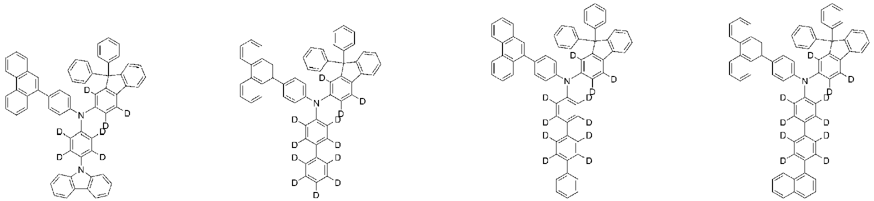

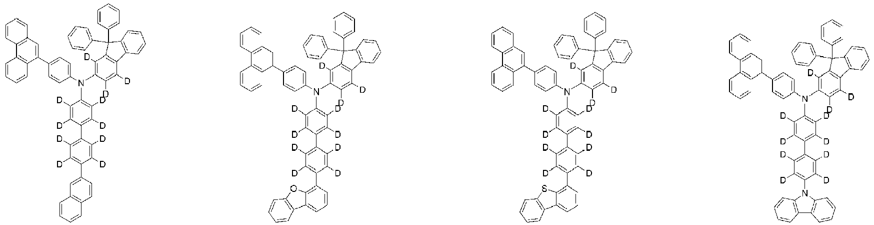

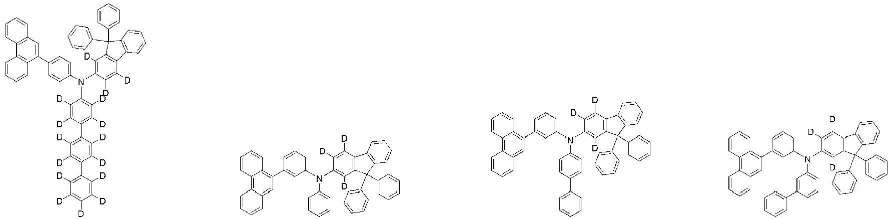

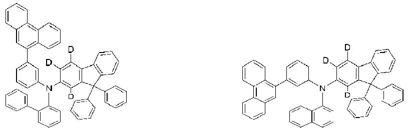

The compound represented by Chemical Formula 1 may be prepared by the preparation method as shown in the following Reaction Scheme 1.

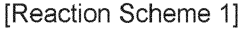

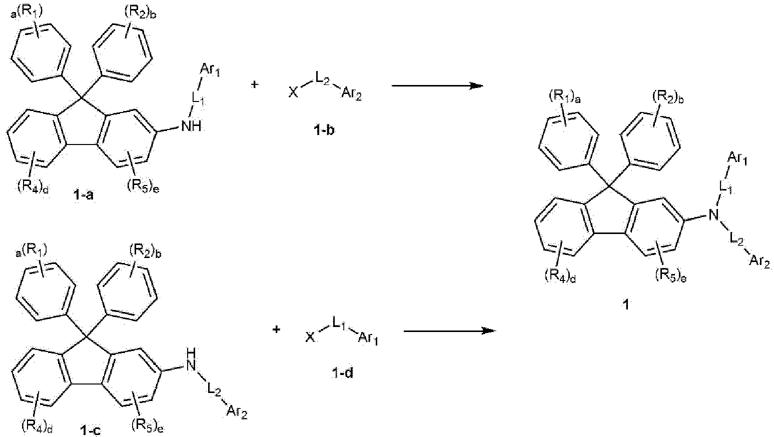

In Reaction Scheme 1, the remainder excluding X is as defined above, and X is halogen, preferably bromo, or chloro. The Reaction Scheme 1 is an amine substitution reaction in which the compound represented by Chemical Formula 1-a is reacted with the compound represented by Chemical Formula 1-b, or the compound represented by Chemical Formula 1-c is reacted with the compound represented by Chemical Formula 1-d, thereby preparing the compound represented by Chemical Formula 1. The reaction is preferably carried out in the presence of a palladium catalyst and a base. The reactive group for the amine substitution reaction can be modified as known in the art. The preparation method can be further specified in Preparation Examples to be described later.

In addition, the present invention provides an organic light emitting device comprising the compound represented by Chemical Formula 1. In one example, the present invention provides an organic light emitting device comprising a first electrode; a second electrode provided at a side opposite to the first electrode; and at least one layer of organic material layers provided between the first electrode and the second electrode, wherein the at least one layer of the organic material layers includes the compound represented by Chemical Formula 1.

The organic material layer of the organic light emitting device of the present invention may have a single layer structure, but it may have a multilayered structure in which two or more organic material layers are stacked. For example, the organic light emitting device of the present invention may have a structure comprising a hole injection layer, a hole transport layer, a hole adjustment layer, a light emitting layer, an electron transport layer, an electron injection layer and the like as the organic material layer. However, the structure of the organic light emitting device is not limited thereto, and it may include a smaller number of organic layers.

Further, the organic material layer may include a hole injection layer, a hole transport layer, a layer simultaneously performing a hole injection and a hole transport, or a hole adjustment layer, wherein the hole injection layer, the hole transport layer, the layer simultaneously performing a hole injection and a hole transport, or a hole adjustment layer include the compound represented by Chemical Formula 1.

Further, the organic material layer may include a light emitting layer, wherein the light emitting layer includes a compound represented by Chemical Formula 1. In particular, the compound according to the present invention can be used as a dopant for the light emitting layer.

Further, the organic material layer may include an electron transport layer or an electron injection layer, wherein the electron transport layer or the electron injection layer includes a compound represented by Chemical Formula 1.

Further, the electron transport layer, the electron injection layer and the layer simultaneously performing an electron transport and an electron injection include a compound represented by Chemical Formula 1.

Further, the organic material layer includes a light emitting layer and an electron transport layer, and the electron transport layer may include a compound represented by Chemical Formula 1.

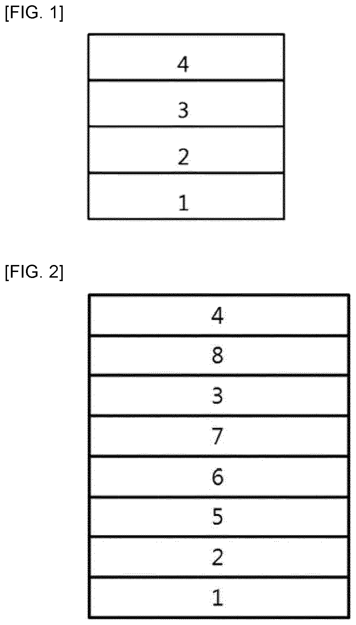

Further, the organic light emitting device according to the present invention may be a normal type organic light emitting device in which an anode, at least one organic material layer, and a cathode are sequentially stacked on a substrate. Further, the organic light emitting device according to the present invention may be an inverted type organic light emitting device in which a cathode, at least one organic material layer and an anode are sequentially stacked on a substrate. For example, the structure of an organic light emitting device according to an embodiment of the present invention is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a hole adjustment layer 7, a light emitting layer 3, an electron transport layer 8, and a cathode 4. In such a structure, the compound represented by Chemical Formula 1 may be included in at least one layer of the hole injection layer, the hole transport layer, the hole adjustment layer, the light emitting layer and the electron transport layer.

The organic light emitting device according to the present invention may be manufactured by materials and methods known in the art, except that at least one layer of the organic material layers includes the compound represented by Chemical Formula 1. In addition, when the organic light emitting device includes a plurality of organic material layers, the organic material layers may be formed of the same material or different materials.

For example, the organic light emitting device according to the present invention can be manufactured by sequentially stacking a first electrode, an organic material layer and a second electrode on a substrate. In this case, the organic light emitting device may be manufactured by depositing a metal, metal oxides having conductivity, or an alloy thereof on the substrate by using a PVD (physical vapor deposition) method such as a sputtering method or an e-beam evaporation method to form the anode, forming an organic material layer including the hole injection layer, the hole transport layer, the light emitting layer, and the electron transport layer thereon, and then depositing a material that can be used as the cathode thereon. In addition to such a method, the organic light emitting device may be manufactured by sequentially depositing a cathode material, an organic material layer and an anode material on a substrate.

In addition, the compound represented by Chemical Formula 1 may be formed into an organic layer by a solution coating method as well as a vacuum deposition method at the time of manufacturing an organic light emitting element. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer, and an anode material on a substrate (International Publication WO 2003/012890). However, the manufacturing method is not limited thereto.

For example, the first electrode is an anode and the second electrode is a cathode, or the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:AI or SNO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has an ability of transporting the holes, thus a hole injecting effect in the anode and an excellent hole injecting effect to the light emitting layer or the light emitting material, prevents movement of an exciton generated in the light emitting layer to the electron injection layer or the electron injection material, and has an excellent thin film forming ability. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The light emitting material is a material capable of emitting light in the visible light region by combining holes and electrons respectively transported from the hole transport layer and the electron transport layer, and having good quantum efficiency for fluorescence or phosphorescence. Specific examples include 8-hydroxy-quinoline aluminum complex (Alq₃); carbazole-based compounds; dimerized styryl compounds; BAIq; 10-hydroxybenzoquinoline-metal compounds; benzoxazole, benzothiazole and benzimidazole-based compunds; poly(p-phenylenevinylene)(PPV)-based polymers; spiro compounds; polyfluorene, rubrene, and the like, but are not limited thereto.

The light emitting layer may include a host material and a dopant material. The host material may be a fused aromatic ring derivative, a heterocycle-containing compound or the like. Specific examples of the fused aromatic ring derivatives include anthracene derivatives, pyrene derivatives, naphthalene derivatives, pentacene derivatives, phenanthrene compounds, fluoranthene compounds, and the like. Examples of heterocyclic compounds include carbazole derivatives, dibenzofuran derivatives, ladder-type furan compounds, pyrimidine derivatives, and the like, but are not limited thereto.

Examples of the dopant material include an aromatic amine derivative, a styrylamine compound, a boron complex, a fluoranthene compound, a metal complex, and the like. Specific examples of the aromatic amine derivatives include substituted or unsubstituted fused aromatic ring derivatives having an arylamino group, and examples thereof include pyrene, anthracene, chrysene, and periflanthene having the arylamino group, and the like. The styrylamine compound is a compound where at least one arylvinyl group is substituted in substituted or unsubstituted arylamine, in which one or more substituents selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

The electron transport layer is a layer receiving the electrons from the electron injection layer and transporting the electrons to the light emitting layer, the electron transport material is a material that can receive the electrons well from the cathode and transport the electrons to the light emitting layer, and a material having large mobility to the electrons is suitable. Specific examples thereof include an 8-hydroxyquinoline Al complex; a complex including Alq₃; an organic radical compound; a hydroxyflavone-metal complex, and the like, but are not limited thereto. The electron transport layer may be used together with a predetermined desired cathode material as used according to the prior art. In particular, an example of an appropriate cathode material is a general material having the low work function and followed by an aluminum layer or a silver layer. Specific examples thereof include cesium, barium, calcium, ytterbium, and samarium, and each case is followed by the aluminum layer or the silver layer.

The electron injection layer is a layer injecting the electrons from the electrode, and a compound which has an ability of transporting the electrons, an electron injecting effect from the cathode, and an excellent electron injecting effect to the light emitting layer or the light emitting material, prevents movement of an exciton generated in the light emitting layer to the hole injection layer, and has an excellent thin film forming ability is preferable. Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone, and the like, and its derivative, a metal complex compound, a nitrogen-containing 5-membered cycle derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present invention may be a front side emission type, a back side light emitting type, or a double side light emitting type according to the used material.

In addition, the compound represented by Chemical Formula 1 may be included in an organic solar cell or an organic transistor in addition to an organic light emitting device.

The preparation of the compound represented by Chemical Formula 1 and the organic light emitting device comprising the same will be described in detail in the following examples. However, these examples are presented for illustrative purposes only, and the scope of the present invention is not limited thereto.

### [Preparation Example 1]

### Preparation Example 1: Preparation of Compounds A1 to A6

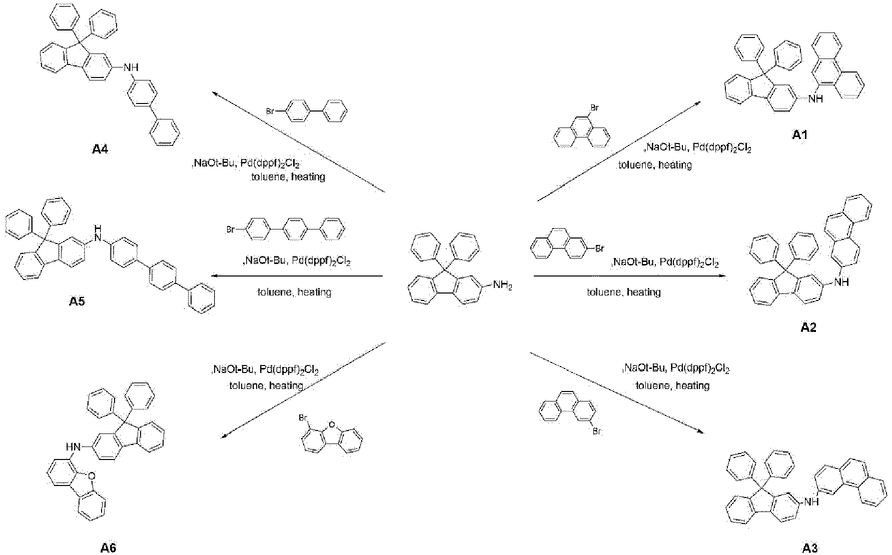

### (Preparation of Compound A1)

9,9-diphenyl-9H-fluoren-2-amine (30 g, 89.97 mmol), 9-bromophenanthrene (23.6 g, 91.77 mmol), and sodium-t-butoxide (12.11 g, 125.9 mol) were added to toluene, and the mixture was heated, stirred and then refluxed. [1,1'-bis(diphenylphosphino)ferrocene]dichloropalladium (658 mg, 1 mol%) was then added thereto. The reaction temperature was lowered to room temperature. After completion of the reaction, recrystallization from tetrahydrofuran and ethyl acetate gave Compound A1 (38.9 g, yield 85%).
MS[M+H]⁺ = 510.65

### (Preparation of Compound A2)

Compound A2 was prepared in the same manner as in Preparation of Compound A1, except that 2-bromophenanthrene was used instead of 9-bromophenanthrene.
MS[M+H]⁺ = 510.65

### (Preparation of Compound A3)

Compound A3 was prepared in the same manner as in Preparation of Compound A1, except that 3-bromophenanthrene was used instead of 9-bromophenanthrene.
MS[M+H]⁺ = 510.65

### (Preparation of Compound A4)

Compound A4 was prepared in the same manner as in Preparation of Compound A1, except that 4-bromobiphenyl was used instead of 9-bromophenanthrene.
MS[M+H]⁺ = 486.63

### (Preparation of Compound A5)

Compound A5 was prepared in the same manner as in Preparation of Compound A1, except that 4-bromo-1,1';4',1"-terphenyl was used instead of 9-bromophenanthrene.
MS[M+H]⁺ = 562.73

### (Preparation of Compound A6)

Compound A6 was prepared in the same manner as in Preparation of Compound A1, except that 4-bromo-dibenzo[b,d]furan was used instead of 9-bromophenanthrene.
MS[M+H]⁺ = 500.61

### Preparation Example 2: Preparation of Compound A7

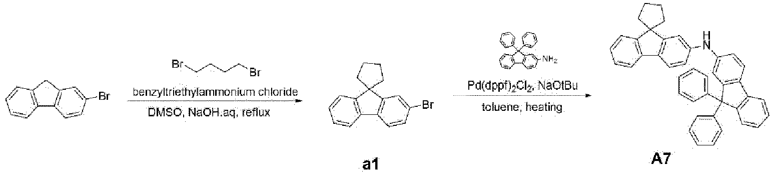

### (Preparation of Compound a1)

2-Bromo-9H-fluorene (100 g, 407.96 mmol) and a 50 wt% aqueous sodium hydroxide solution (70 g, 897.51 mmol) were added to dimethylsulfoxide (1000 ml) to which 1,4-dibromobutane (88.09 g, 407.96 mmol) was added dropwise at 160°C, and then heated and stirred for 3 hours. The reaction temperature was lowered to room temperature. After completion of the reaction, the reaction mixture was extracted with toluene and water, and then purified by column chromatography to give Compound A1 (ivory liquid, 120 g, yield 98%).
MS[M+H]⁺ = 299.14

### (Preparation of Compound A7)

Compound A1 (10 g, 33.55 mmol), 9,9'-diphenyl-9H-fluoren-2-amine (11.29 g, 33.89 mmol) and sodium-t-butoxide (4.5 g, 46.97 mol) were added to toluene, and the mixture was heated, stirred and then refluxed. [1,1'-Bis(diphenylphosphino)ferrocene]dichloropalladium (490 mg, 2 mmol%) was then added thereto. The reaction temperature was then lowered to room temperature. After completion of the reaction, recrystallization from chloroform and ethyl acetate gave Compound A2.
MS[M+H]⁺= 552.26

### Preparation Example 3: Preparation of Compounds B1 to B4

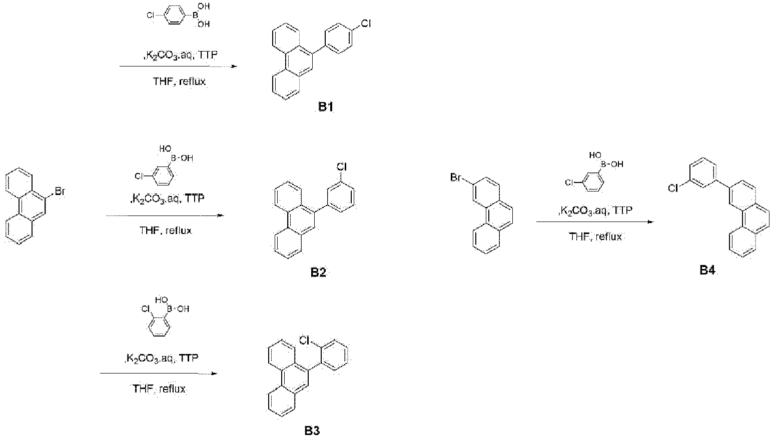

### (Preparation of Compound B1)

9-Bromophenanthrene (50 g, 194.4 mmol) and 4-chlorophenylboronic acid (31.93 g, 204.1 mmol) were added to tetrahydrofuran (300 ml). Then, 2M aqueous potassium carbonate solution (150 ml) was added and tetrakistriphenylphosphinopalladium (4.5g, 2 mol%) was added, and the mixture was heated and stirred for 3 hours. The reaction temperature was lowered to room temperature. After completion of the reaction, the aqueous potassium carbonate solution was removed and the layers were separated. After removal of the solvent, the white solid was recrystallized from ethyl acetate to give Compound B1 (50.53 g, yield 90%).
MS[M+H]⁺ = 289.77

### (Preparation of Compound B2)

Compound B2 was prepared in the same manner as in Preparation of Compound B1, except that 3-chlorophenylboronic acid was used instead of 4-chlorophenylboronic acid.
MS[M+H]⁺ = 289.77

### (Preparation of Compound B3)

Compound B3 was prepared in the same manner as in Preparation of Compound B1, except that 2-chlorophenylboronic acid was used instead of 4-chlorophenylboronic acid.
MS[M+H]⁺ = 289.77

### (Preparation of Compound B4)

Compound B2 was prepared in the same manner as in Preparation of Compound B1, except that 2-bromophenanthrene was used instead of 9-bromophenanthrene, and 3-chlorophenylboronic acid was used instead of 4-chlorophenylboronic acid.
MS[M+H]⁺ = 289.77

### [Example]

### Example 1: Preparation of Compound 1

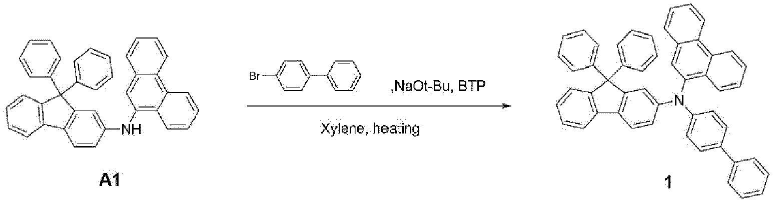

Compound A1 (15 g, 29.43 mmol), 4-bromobiphenyl (7 g, 30.02 mmol) and sodium-t-butoxide (3.9 g, 41.2 mol) were added to xylene, and the mixture was heated, stirred and then refluxed. Bis(tri-t-butylphosphine)]palladium (170 mg, 1 mmol%) was then added thereto. The reaction temperature was lowered to room temperature. After completion of the reaction, recrystallization from tetrahydrofuran and ethyl acetate gave Compound 1 (19.48 g, yield 75%).
MS[M+H]⁺ = 662.85

### Example 2: Preparation of Compound 2

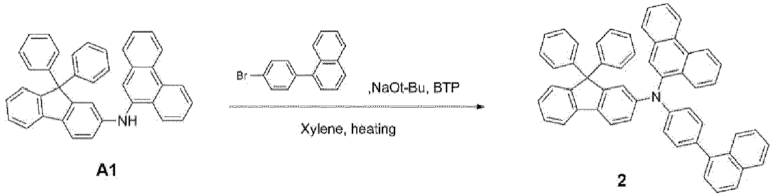

Compound 2 was prepared in the same manner as in Preparation of Compound 1, except that 1-(4-bromophenyl)naphthalene was used instead of 4-bromobiphenyl.
MS[M+H]⁺ = 712.91

### Example 3: Preparation of Compound 3

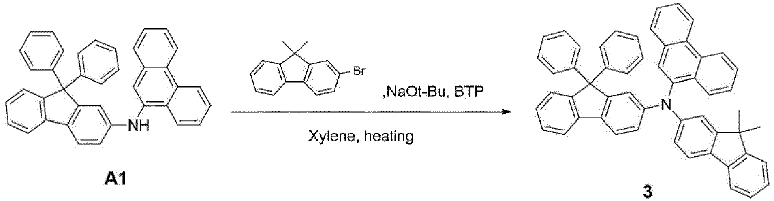

Compound 3 was prepared in the same manner as in Preparation of Compound 1, except that 2-bromo-9,9-dimethyl-9H-fluorene was used instead of 4-bromobiphenyl.
MS[M+H]⁺ = 702.91

### Example 4: Preparation of Compound 4

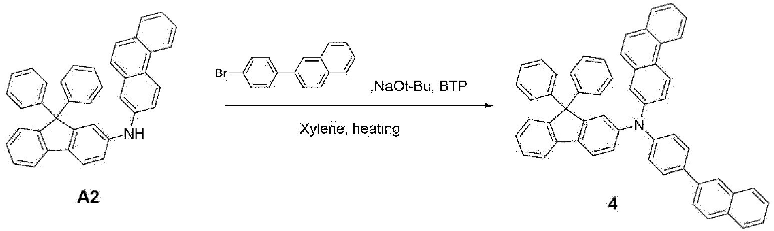

Compound 4 was prepared in the same manner as in Preparation of Compound 1, except that Compound A2 was used instead of Compound A1, and 2-(4-bromophenyl)naphthalene was used instead of 4-bromobiphenyl.
MS[M+H]⁺ = 712.91

### Example 5: Preparation of Compound 5

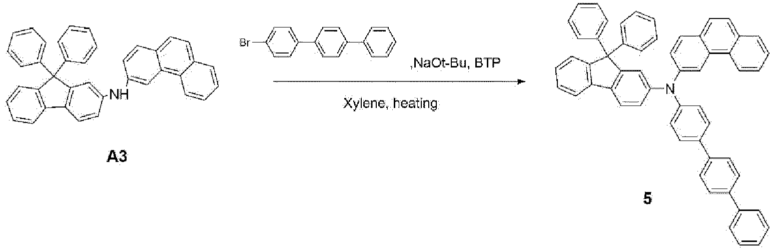

Compound 5 was prepared in the same manner as in Preparation of Compound 1, except that Compound A3 was used instead of Compound A1, and 4-bromo-1,1';4',1"-terphenyl was used instead of 4-bromobiphenyl.
MS[M+H]⁺ = 738.95

### Example 6: Preparation of Compound 6

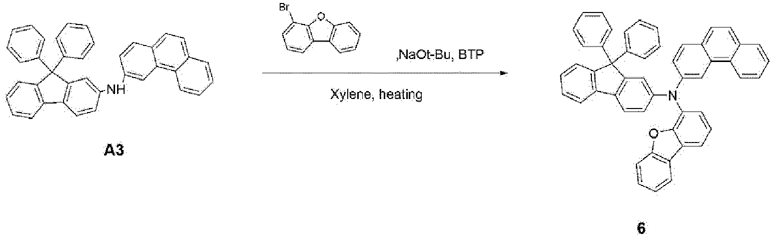

Compound 6 was prepared in the same manner as in Preparation of Compound 1, except that Compound A3 was used instead of Compound A1, and 4-bromo-dibenzo[b,d]furan was used instead of 4-bromobiphenyl.
MS[M+H]⁺ = 676.83

### Example 7: Preparation of Compound 7

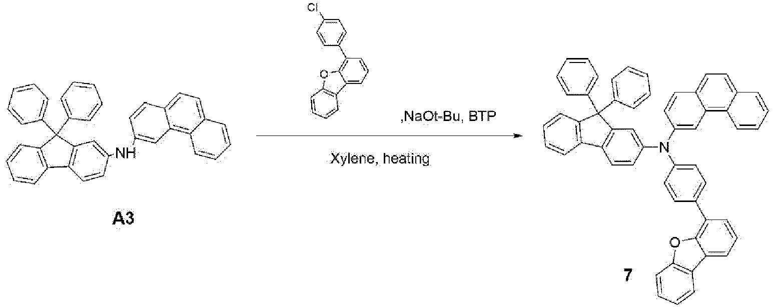

Compound 7 was prepared in the same manner as in Preparation of Compound 1, except that Compound A3 was used instead of Compound A1, and 4-(4-chlorophenyl)dibenzo[b,d]furan was used instead of 4-bromobiphenyl.
MS[M+H]⁺ = 752.93

### Example 8: Preparation of Compound 8

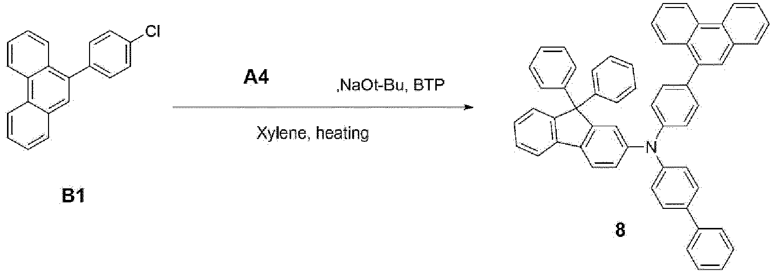

Compound 8 was prepared in the same manner as in Preparation of Compound 1, except that Compound B1 was used instead of Compound A1, and Compound A4 was used instead of 4-bromobiphenyl.
MS[M+H]⁺ = 738.95

### Example 9: Preparation of Compound 9

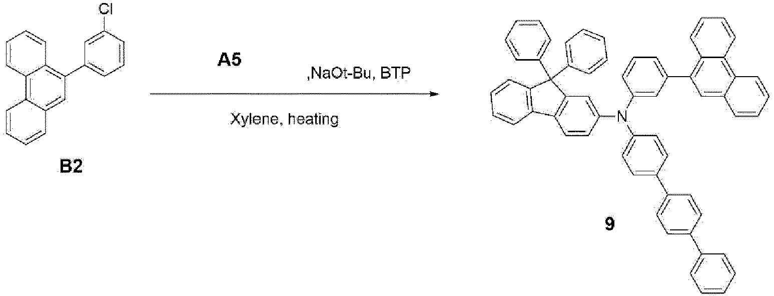

Compound 9 was prepared in the same manner as in Preparation of Compound 1, except that Compound B2 was used instead of Compound A1, and Compound A5 was used instead of 4-bromobiphenyl.
MS[M+H]⁺ = 815.04

### Example 10: Preparation of Compound 10

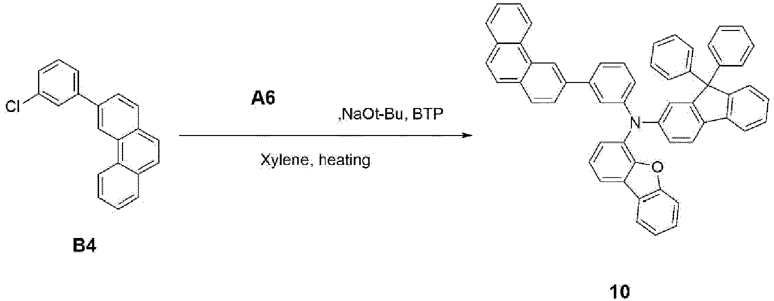

Compound 10 was prepared in the same manner as in Preparation of Compound 1, except that Compound B3 was used instead of Compound A1, and Compound A6 was used instead of 4-bromobiphenyl.
MS[M+H]⁺ = 752.93

### Example 11: Preparation of Compound 11

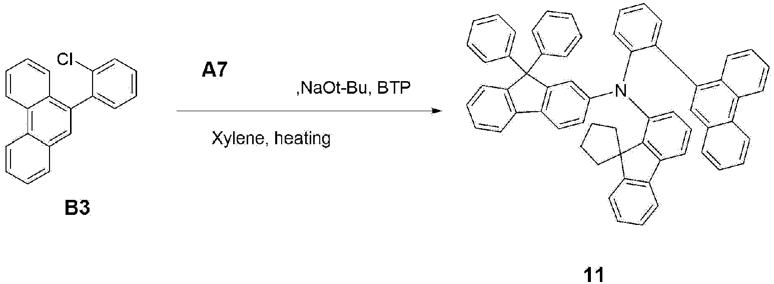

Compound 11 was prepared in the same manner as in Preparation of Compound 1, except that Compound B3 was used instead of Compound A1, and Compound A7 was used instead of 4-bromobiphenyl.
MS[M+H]⁺ = 804.36

### Example 12: Preparation of Compound 12

### Step 1) Preparation of Compound 1-A

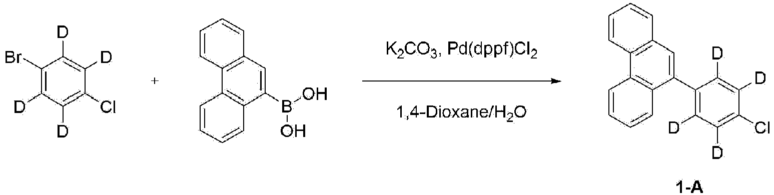

Bromo-4-chlorobenzene-2,3,5,6-d₄ (50.00 g, 255.78 mmol) and phenanthrene-9-ylboronic acid (59.64 g, 268.57 mmol) were dissolved in 1,4-dioxane (600 ml) to which a solution of potassium carbonate (106.05 g, 767.34 mmol: water 300 ml) was added and then heated and stirred for 10 minutes. To the solution, 1,1'-bis(diphenylphosphino)ferrocene dichloropalladium(II) (0.56 g, 0.77 mmol) dissolved in 1,4-dioxane (20 ml) was added, and the mixture was heated and stirred for 1 hour. After completion of the reaction, the mixture was filtered and the layers were separated from chloroform and water. After removal of the solvent, recrystallization from hexane gave Compound 1-A (58.5 g, yield 78.11%).

### Step 2) Preparation of Compound 12

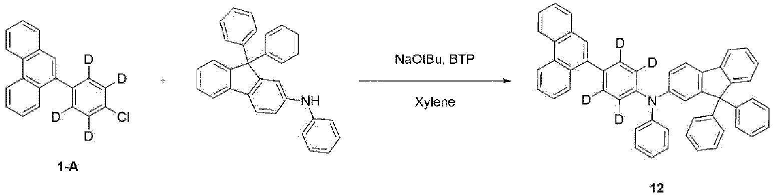

Xylene (250 ml) was added to the above prepared Compound 1-A (20.0 g, 68.31 mmol), N-9,9-triphenyl-9H-fluoren-2-amine (28.53 g, 69.67 mmol), and sodium tert-butoxide (9.19 g, 95.63 mmol), and then heated and stirred for 10 minutes. To the mixture, bis(tri-tert-butylphosphine)palladium (0.10 g, 0.20 mmol) dissolved in xylene (20 ml) was added, and then heated and stirred for 1 hour. After completion of the reaction, the mixture was filtered and the layers were separated from toluene and water. After removal of the solvent, recrystallization from ethyl acetate gave Compound 12 (35.5 g, yield 78.05%).
MS[M+H]⁺ = 666

### Example 13: Preparation of Compound 13

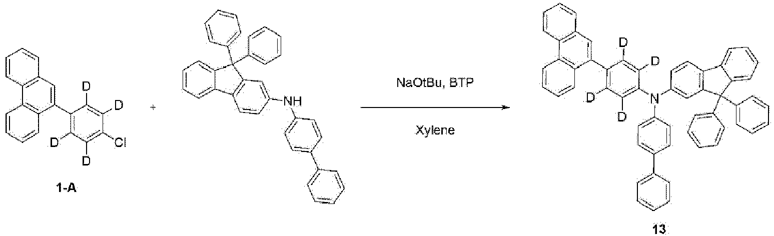

Compound 13 (38.3 g, yield 75.57%) was prepared in the same manner as in Step 2 of Example 12, except that Compound 1-A (20.0 g, 68.31 mmol) prepared in Example 12, and N-([1,1'-biphenyl]-4-yl)-9,9-diphenyl] -9H-fluoren-2-amine (33.83 g, 69.67 mmol) were used.
MS[M+H]⁺ = 742

### Example 14: Preparation of Compound 14

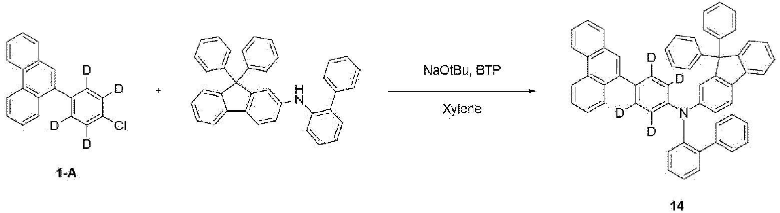

Compound 14 (37.2 g, yield 73.40%) was prepared in the same manner as in Step 2 of Example 12, except that Compound 1-A (20.0 g, 68.31 mmol) prepared in Step 1 of Example 12, and N-([1,1'-biphenyl]-2-yl)-9,9-diphenyl]-9H-fluoren-2-amine (33.83 g, 69.67 mmol) were used.
MS[M+H]⁺ = 742

### Example 15: Preparation of Compound 15

### Step 1): Preparation of Compound 4-A

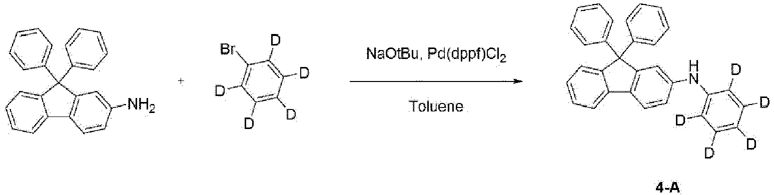

Toluene (400 ml) was added to 9,9-diphenyl-9H-fluoren-2-amine (50.0 g, 149.96 mmol), 1-bromobenzene-2,3,4,5,6-d₅ (24.30 g, 149.96 mmol), and sodium tert-butoxide (20.18 g, 209.94 mmol), and then heated and stirred. To the mixture, 1,1'-bis(diphenylphosphino)ferrocene dichloropalladium(II) (0.55 g, 0.75 mmol) dissolved in toluene (20 ml) was added, and then heated and stirred for 1 hour. After completion of the reaction, the mixture was filtered, and the layers were separated from toluene and water. After removal of the solvent, recrystallization from ethyl acetate gave Compound 4-A (50.1 g, yield 80.59%).

### Step 2): Preparation of Compound 15

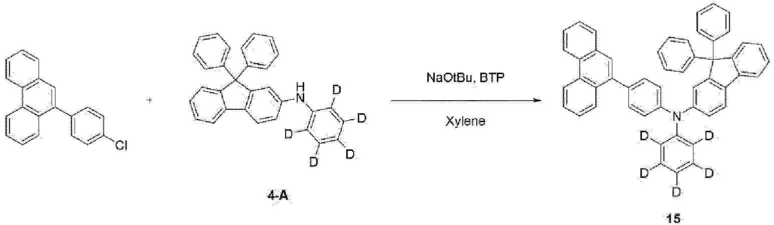

Compound 15 (34.5 g, yield 74.70%) was prepared in the same manner as in Step 2 of Example 12, except that 9-(4-chlorophenyl)phenanthrene (20.0 g, 69.26 mmol) and the above prepared Compound 4-A (29.29 g, 70.64 mmol) were used.
MS[M+H]⁺ = 667

### Example 16: Preparation of Compound 16

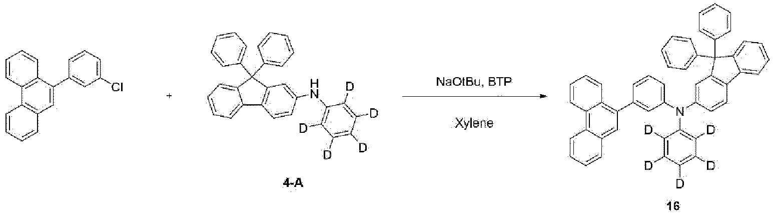

Compound 16 (33.3 g, yield 72.12%) was prepared in the same manner as in Step 2 of Example 12, except that 9-(3-chlorophenyl)phenanthrene (20.0 g, 69.26 mmol) and Compound 4-A (29.29 g, 70.64 mmol) prepared in Step 1 of Example 15 were used.
MS[M+H]⁺ = 667

### Example 17: Preparation of Compound 17

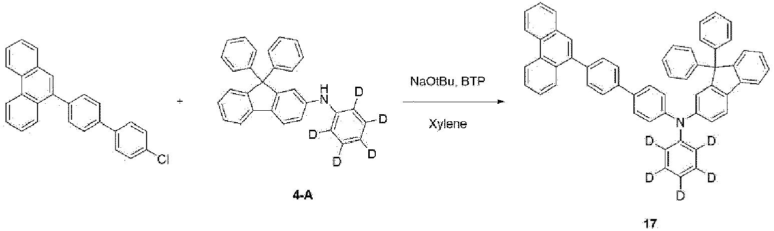

Compound 17 (30.5 g, yield 74.90%) was prepared in the same manner as in Step 2 of Example 12, except that 9-(4'-chloro[1,1'-biphenyl]-4-yl)phenanthrene (20.0 g, 54.81 mmol) and Compound 4-A (23.18 g, 55.91 mmol) prepared in Step 1 of Example 15 were used.
MS[M+H]⁺ = 743

### Example 18: Preparation of Compound 18

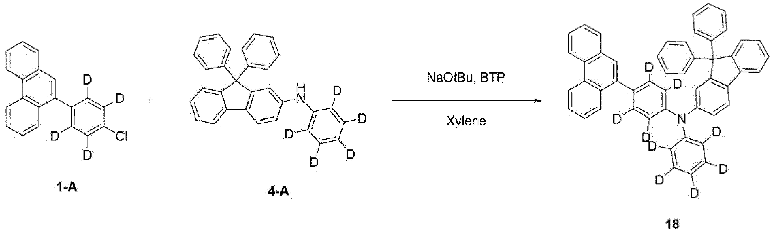

Compound 18 (34.9 g, yield 76.15%) was prepared in the same manner as in Step 2 of Example 12, except that Compound 1-A (20.0 g, 68.31 mmol) prepared in Step 1 of Example 12 and Compound 4-A (28.88 g, 69.67 mmol) prepared in Step 1 of Example 15 were used.
MS[M+H]⁺ = 671

### Example 19: Preparation of Compound 19

### Step 1) Preparation of Compound 8-A

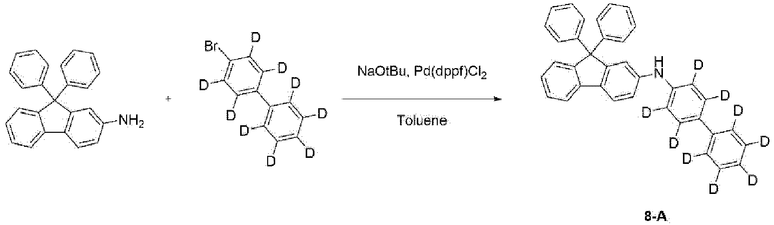

Compound 8-A (58.3 g, yield 78.59%) was prepared in the same manner as in Step 2 of Example 15, except that 9,9-diphenyl-9H-fluoren-2-amine (50.0 g, 149.96 mmol) and 4-bromo-biphenyl-2,2',3,3',4',5,5',6,6'-d₉ (36.31 g, 149.96 mmol) were used.

### Step 2) Preparation of Compound 19

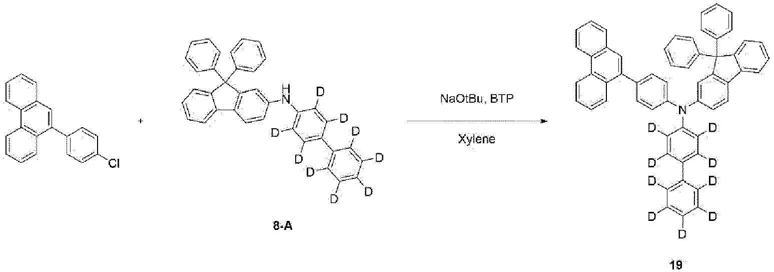

Compound 19 (38.1 g, yield 74.14%) was prepared in the same manner as in Step 2 of Example 12, except that 9-(4-chlorophenyl)phenanthrene (20.0 g, 69.26 mmol) and the above prepared Compound 8-A (34.94 g, 70.64 mmol) were used.
MS[M+H]⁺ = 747

### Example 20: Preparation of Compound 20

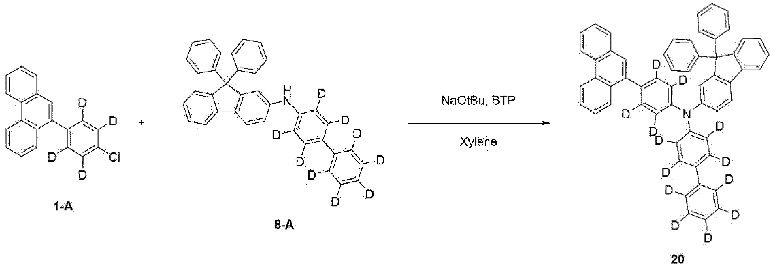

Compound 20 (36.7 g, yield 71.54%) was prepared in the same manner as in Step 2 of Example 12, except that Compound 1-A (20.0 g, 68.31 mmol) prepared in Step 1 of Example 12 and Compound 8-A (34.41 g, 69.67 mmol) prepared in Step 1 of Example 15 were used.
MS[M+H]⁺ = 751

### Example 21: Preparation of Compound 21

### Step 1) Preparation of Compound 10-A

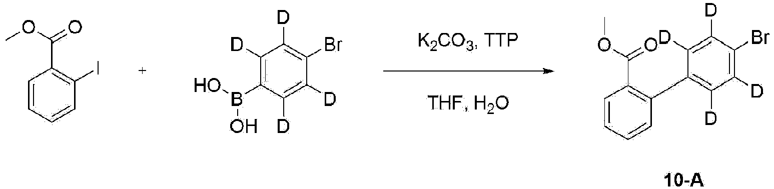

Methyl-2-iodobenzoate (50.0 g, 190.80 mmol), (4-bromophenyl-2,3,5,6-d4)boronic acid (41.04 g, 200.34 mmol) were dissolved in tetrahydrofuran (3000 ml) to which a solution of potassium carbonate (79.11 g, 572.40 mmol: water 200 ml) was added, and then heated and stirred for 10 minutes. To the solution, tetrakis(triphenylphosphine)palladium(0) (1.10 g, 0.95 mmol) dissolved in tetrahydrofuran (20 ml) was added, and then heated and stirred for 1 hour. After completion of the reaction, the mixture was filtered and the layers were separated from chloroform and water. After removal of the solvent, recrystallization from hexane gave Compound 10-A (43.0 g, yield 76.35%).

### Step 2) Preparation of Compound 10-B

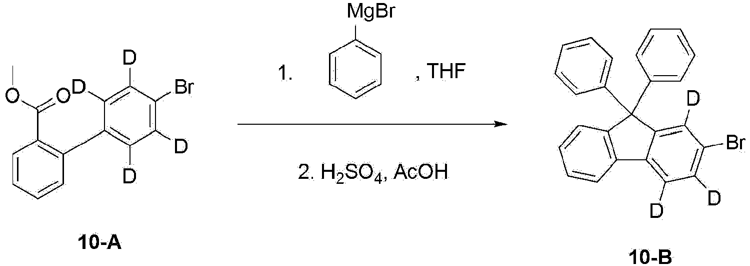

The above prepared Compound 10-A (43.0 g, 145.68 mmol) was dissolved in tetrahydrofuran (300 ml), and the temperature was lowered to -78°C. To the solution, phenylmagnesium bromide (55.47 g, 305.93 mmol) was added, and then stirred for 30 minutes. After completion of the reaction, 1N hydrochloric acid (300 ml) was added thereto and the layers were separated. After removal of the solvent, acetic acid (300 ml) was added, refluxed and stirred, and then sulfuric acid (cat.) was added dropwise. After completion of the reaction, the mixture was filtered and the layers were separated from chloroform and aqueous sodium hydrogen carbonate solution. After removal of the solvent, recrystallization from hexane gave Compound 10-B (40.0 g, yield 68.59%).

### Step 3) Preparation of Compound 10-C

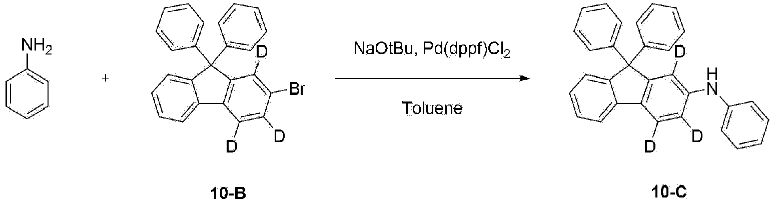

Compound 10-C (40.0 g, yield 90.29%) was prepared in the same manner as in Step 1 of Example 15, except that aniline (10.0 g, 107.38 mmol) and the above prepared Compound 10-B (42.99 g, 149.96 mmol) were used.

### Step 4) Preparation of Compound 21

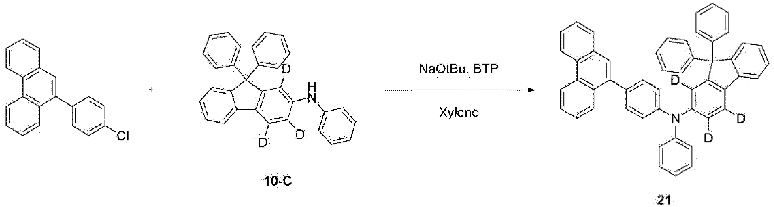

Compound 21 (32.5 g, yield 70.58%) was prepared in the same manner as in Step 2 of Example 12, except that 9-(4-chlorophenyl)phenanthrene (20.0 g, 69.26 mmol) and the above prepared Compound 10-C (29.14 g, 70.64 mmol) were used.
MS[M+H]⁺ = 665

### Example 22: Preparation of Compound 22

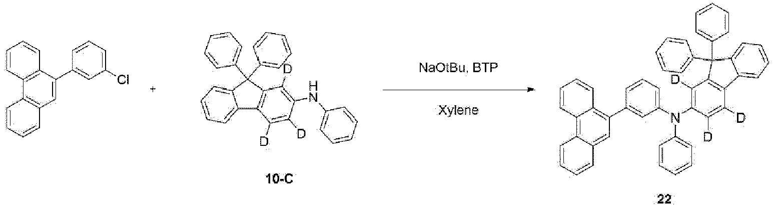

Compound 22 (32.0 g, yield 69.49%) was prepared in the same manner as in Step 2 of Example 12, except that 9-(3-chlorophenyl)phenanthrene (20.0 g, 69.26 mmol) and Compound 10-C (29.14 g, 70.64 mmol) prepared in Step 2 of Example 12 were used.
MS[M+H]⁺ = 665

### [Experimental Example]

### Experimental Example 1-1

A glass substrate (Corning 7059 glass) on which a thin film of ITO (indium tin oxide) was coated in a thickness of 1,000 Å was put into distilled water in which a detergent was dissolved, and ultrasonically washed. In this case, a product manufactured by Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted sequentially using isopropyl alcohol, acetone, and methanol solvents, and drying was conducted.

On the ITO transparent electrode thus prepared, the following Compound HI-1 was thermally vacuum-deposited in a thickness of 500 Å to form a hole injection layer. The previously prepared Compound 1 was vacuum deposited in a thickness of 900 Å on the hole injection layer to form a hole transport layer. Subsequently, the following Compound HT2 was vacuum deposited in a thickness of 50 Å on the hole transport layer to form a hole adjustment layer. The following Compound H1 and the following Compound D1 were vacuum deposited at a weight ratio of 25: 1 in a thickness of 300 Å on the hole adjustment layer to form a light emitting layer. The following Compound E1 was vacuum deposited in a thickness of 300 Å on the light emitting layer to form an electron transport layer. Lithium fluoride (LiF) with a thickness of 12Å and aluminum with a thickness of 2,000 Å were sequentially deposited on the electron transport layer to form a cathode, thereby manufacturing an organic light emitting device.

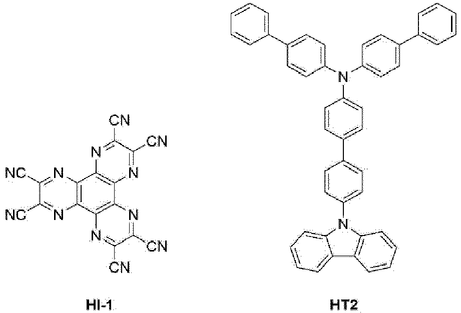

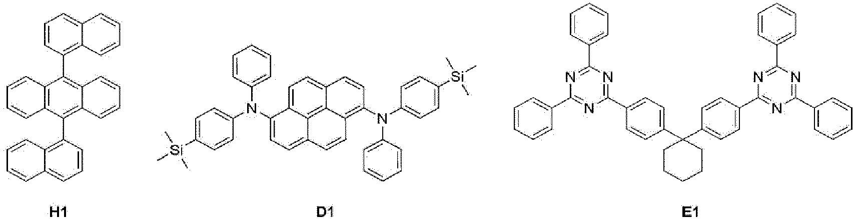

In the aforementioned procedure, the vapor deposition rate of the organic material was maintained at 1 Å/sec, the vapor deposition rate of lithium fluoride was maintained at 0.2 Å/sec, and the vapor deposition rate of aluminum was maintained at 3 to 7 Å/sec.

### Experimental Examples 1-2 to 1-4

The organic light emitting devices were manufactured in the same manner as in Experimental Example 1-1, except that Compounds shown in Table 1 below were used instead of Compound 1 as a hole transport layer.

### Comparative Experimental Examples 1-1 to 1-5

The organic light emitting devices were manufactured in the same manner as in Experimental Example 1-1, except that Compounds shown in Table 1 below were used instead of Compound 1 as a hole transport layer. In Table 1, Compounds HT1, HT3, HT4, HT7, and HT8 are as follows:

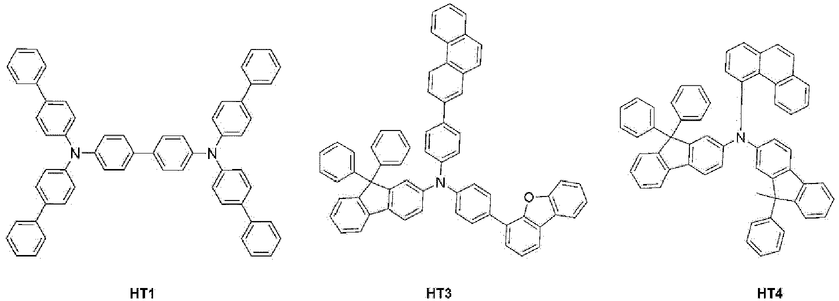

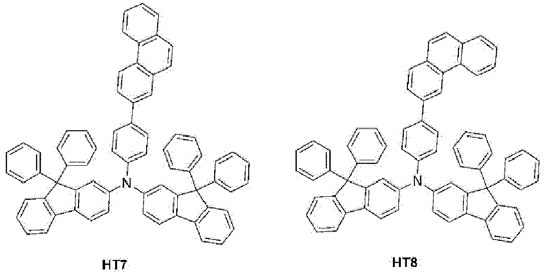

The voltages, efficiencies, color coordinates, and lifetimes were measured by applying a current (20 mA/cm²) to the organic light emitting devices manufactured in Experimental Examples and Comparative Experimental Examples, and the results are shown in Table 1 below. T95 means the time taken for the luminance to be reduced to 95% of the initial luminance.

**[Table 1]**

| | Hole transport layer | Hole adjustment layer | Voltage (V) | Efficiency (cd/A) | color coordinate (x,y) | T95 (hr) |
|---|---|---|---|---|---|---|
| Experimental Ex. 1-1 | Com.1 | HT2 | 3.51 | 6.71 | (0.135, 0.138) | 49.0 |
| Experimental Ex. 1-2 | Com.2 | HT2 | 3.45 | 6.63 | (0.134, 0.137) | 50.2 |
| Experimental Ex. 1-3 | Com.3 | HT2 | 3.41 | 6.58 | (0.135, 0.138) | 55.2 |
| Experimental Ex. 1-4 | Com.6 | HT2 | 3.34 | 6.82 | (0.134, 0.138) | 51.2 |
| Comparative Experimental Ex. 1-1 | HT1 | HT2 | 3.82 | 5.70 | (0.134, 0.139) | 28.1 |
| Comparative Experimental Ex. 1-2 | HT3 | HT2 | 3.94 | 5.81 | (0.135, 0.138) | 21.0 |
| Comparative Experimental Ex. 1-3 | HT4 | HT2 | 3.78 | 5.66 | (0.134, 0.138) | 33.0 |
| Comparative Experimental Ex. 1-4 | HT7 | HT2 | 4.02 | 5.31 | (0.135, 0.138) | 23.1 |
| Comparative Experimental Ex. 1-5 | HT8 | HT2 | 3.89 | 5.51 | (0.134, 0.138) | 25.4 |

### Experimental Example 2-1

A glass substrate (Corning 7059 glass) on which a thin film of ITO (indium tin oxide) was coated in a thickness of 1,000 Å was put into distilled water in which a detergent was dissolved, and ultrasonically washed. In this case, a product manufactured by Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted sequentially using isopropyl alcohol, acetone, and methanol solvents, and drying was conducted.

On the ITO transparent electrode thus prepared, the following Compound HI-1 was thermally vacuum-deposited in a thickness of 500 Å to form a hole injection layer. The following Compound HT1 was vacuum deposited in a thickness of 900 Å thereon to form a hole transport layer. Subsequently, the previously prepared Compound 1 was vacuum deposited in a thickness of 50 Å on the hole transport layer to form a hole adjustment layer. The following Compound H1 and the following Compound D1 were vacuum deposited at a weight ratio of 25: 1 in a thickness of 300 Å on the hole adjustment layer to form a light emitting layer. The following Compound E1 was vacuum deposited in a thickness of 300 Å on the light emitting layer to form an electron transport layer. Lithium fluoride (LiF) with a thickness of 12 Å and aluminum with a thickness of 2,000 Å were sequentially deposited on the electron transport layer to form a cathode, thereby manufacturing an organic light emitting device.

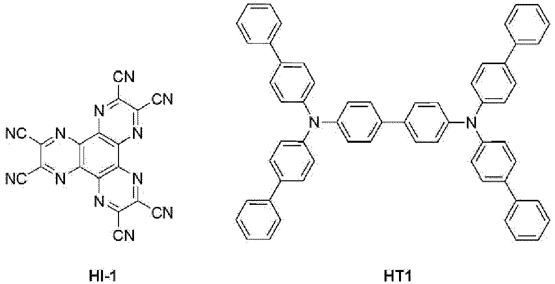

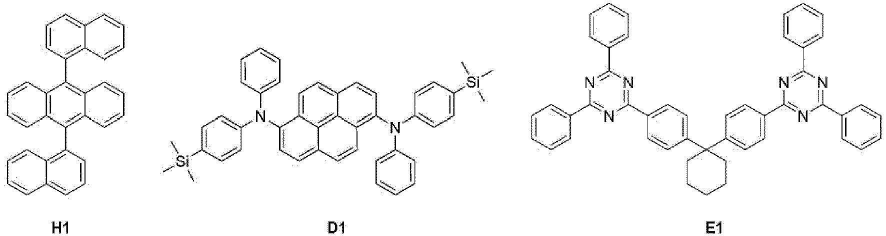

In the aforementioned procedure, the vapor deposition rate of the organic material was maintained at 1 Å/sec, the vapor deposition rate of lithium fluoride was maintained at 0.2 Å/sec, and the vapor deposition rate of aluminum was maintained at 3 to 7 Å/sec.

### Experimental Examples 2-2 to 2-24

The organic light emitting devices were manufactured in the same manner as in Experimental Example 2-1, except that Compounds shown in Table 2 below were used as a hole transport layer and a hole adjustment layer.

### Comparative Experimental Examples 2-1 to 2-6

The organic light emitting devices were manufactured in the same manner as in Experimental Example 2-1, except that Compounds shown in Table 2 below were used as a hole transport layer. In Table 2, Compounds HT3, HT4, HT5, HT7, and HT8 are as follows:

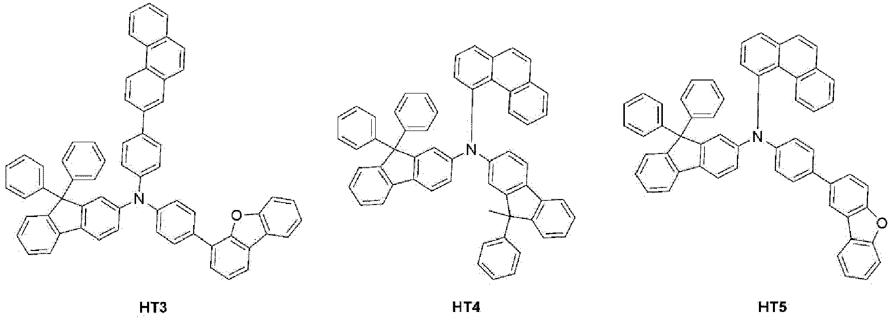

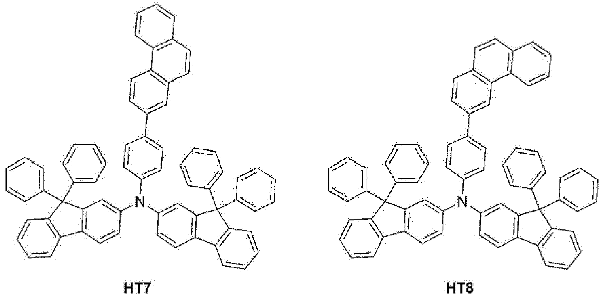

The voltages, efficiencies, color coordinates, and lifetimes were measured by applying a current (20 mA/cm²) to the organic light emitting devices manufactured in Experimental Examples and Comparative Experimental Examples, and the results are shown in Table 2 below. T95 means the time taken for the luminance to be reduced to 95% of the initial luminance.

**[Table 2]**

| | Hole transport layer | Hole adjustment layer | Voltage (V) | Efficiency (cd/A) | Color coordinate (x,y) | T95 (hr) |
|---|---|---|---|---|---|---|
| Experimental Ex. 2-1 | HT1 | Com. 1 | 3.33 | 6.89 | (0.135, 0.138) | 52.0 |
| Experimental Ex. 2-2 | HT1 | Com. 2 | 3.52 | 6.79 | (0.134, 0.138) | 48.0 |
| Experimental Ex. 2-3 | HT1 | Com. 4 | 3.44 | 6.67 | (0.134, 0.138) | 46.8 |
| Experimental Ex. 2-4 | HT1 | Com. 5 | 3.45 | 6.58 | (0.137, 0.134) | 47.1 |
| Experimental Ex. 2-5 | HT1 | Com. 7 | 3.52 | 6.87 | (0.138, 0.138) | 42.5 |
| Experimental Ex. 2-6 | HT1 | Com. 8 | 3.38 | 6.82 | (0.135, 0.139) | 46.5 |
| Experimental Ex. 2-7 | HT1 | Com. 9 | 3.39 | 6.81 | (0.135, 0.138) | 49.7 |
| Experimental Ex. 2-8 | HT1 | Com. 10 | 3.51 | 6.71 | (0.135, 0.139) | 50.1 |
| Experimental Ex. 2-9 | HT1 | Com. 11 | 3.45 | 6.63 | (0.134, 0.138) | 49.8 |
| Experimental Ex. 2-10 | Com. 3 | Com. 8 | 3.41 | 6.58 | (0.134, 0.138) | 47.4 |
| Experimental Ex. 2-11 | Com. 6 | Com. 10 | 3.41 | 6.41 | (0.134, 0.138) | 47.6 |
| Experimental Ex. 2-12 | HT1 | Com. 12 | 3.38 | 6.61 | (0.134, 0.138) | 50.2 |
| Experimental Ex. 2-13 | HT1 | Com. 13 | 3.42 | 6.55 | (0.134, 0.138) | 53.5 |
| Experimental Ex. 2-14 | HT1 | Com. 14 | 3.45 | 6.52 | (0.135, 0.137) | 49.2 |
| Experimental Ex. 2-15 | HT1 | Com. 15 | 3.39 | 6.49 | (0.134, 0.138) | 48.1 |
| Experimental Ex. 2-16 | HT1 | Com. 16 | 3.40 | 6.61 | (0.134, 0.138) | 44.9 |
| Experimental Ex. 2-17 | HT1 | Com. 17 | 3.41 | 6.68 | (0.134, 0.138) | 52.1 |
| Experimental Ex. 2-18 | HT1 | Com. 18 | 3.41 | 6.55 | (0.134, 0.138) | 53.1 |
| Experimental Ex. 2-19 | HT1 | Com. 19 | 3.52 | 6.48 | (0.134, 0.138) | 50.4 |
| Experimental Ex. 2-20 | HT1 | Com. 20 | 3.38 | 6.51 | (0.134, 0.138) | 48.9 |
| Experimental Ex. 2-21 | HT1 | Com. 21 | 3.31 | 6.66 | (0.135, 0.137) | 49.5 |
| Experimental Ex. 2-22 | HT1 | Com. 22 | 3.42 | 6.52 | (0.134, 0.138) | 47.5 |
| Experimental Ex. 2-23 | Com. 16 | Com. 12 | 3.44 | 6.80 | (0.134, 0.138) | 55.1 |
| Experimental Ex. 2-24 | Com. 2 | Com. 18 | 3.44 | 6.78 | (0.135, 0.137) | 54.1 |
| Comparative Experimental Ex. 2-1 | HT1 | HT3 | 3.82 | 5.71 | (0.134, 0.138) | 33.5 |
| Comparative Experimental Ex. 2-2 | HT1 | HT4 | 3.78 | 5.89 | (0.137, 0.135) | 28.2 |
| Comparative Experimental Ex. 2-3 | HT1 | HT5 | 3.75 | 5.91 | (0.134, 0.138) | 35.1 |
| Comparative Experimental Ex. 2-4 | HT4 | HT5 | 3.70 | 5.84 | (0.135, 0.137) | 29.4 |
| Comparative Experimental Ex. 2-5 | HT1 | HT7 | 3.80 | 5.78 | (0.134, 0.138) | 33.5 |
| Comparative Experimental Ex. 2-6 | HT1 | HT8 | 3.82 | 5.94 | (0.135, 0.137) | 34.8 |

As shown in Tables 1 and 2 above, the compounds according to the present invention can play a role of hole transport and hole adjustment in organic electronic devices including organic light emitting devices, and the devices according to the present invention can exhibit excellent characteristics in terms of efficiency, driving voltage, and stability.

**[Description of symbols]**

| | |
|---|---|
| 1: substrate | 2: anode |
| 3: light emitting layer | 4: cathode |
| 5: hole injection layer | 6: hole transport layer |
| 7: hole adjustment layer | 8: electron transport layer |

## Claims

1. A compound represented by the following Chemical Formula 1: 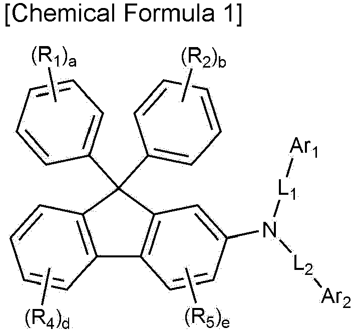 in Chemical Formula 1,
R₁, R₂, R₄ and R₅ are each independently hydrogen, deuterium, halogen, cyano, a substituted or unsubstituted C₁₋₆₀ alkyl, a substituted or unsubstituted C₁₋₆₀ alkoxy, a substituted or unsubstituted C₁₋₆₀ thioalkyl, a substituted or unsubstituted C₃₋₆₀ cycloalkyl, a substituted or unsubstituted C₆₋₆₀ aryl, or a tri(C₁₋₆₀ alkyl)silyl,
a, b and d are each independently an integer of 0 to 4,
e is an integer of 0 to 3,
L₁ and L₂ are each independently a single bond; a substituted or unsubstituted C₆₋₆₀ arylene; or a substituted or unsubstituted C₂₋₆₀ heteroarylene containing at least one hetero atom selected from the group consisting of N, O, and S, and
Ar₁ is represented by the following Chemical Formula 2, 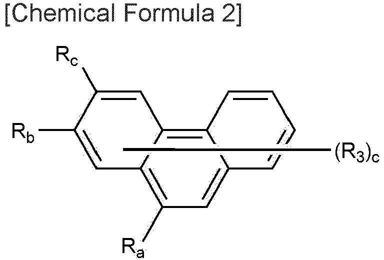
in Chemical Formula 2,
one of Rₐ, R_{b} and R_{c} is bonded to L₁ and the rest are hydrogen,
R₃ is hydrogen, deuterium, halogen, cyano, a substituted or unsubstituted C₁₋₆₀ alkyl, a substituted or unsubstituted C₁₋₆₀ alkoxy, a substituted or unsubstituted C₁₋₆₀ thioalkyl, a substituted or unsubstituted C₃₋₆₀ cycloalkyl, a substituted or unsubstituted C₆₋₆₀ aryl, or a tri(C₁₋₆₀ alkyl)silyl,
c is an integer of 0 to 7, and
Ar₂ is any one aryl selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b; or any one heteroaryl selected from the group consisting of dibenzofuranyl and dibenzothiophenyl, wherein the Ar₂ is unsubstituted or substituted with one to five deuterium atoms, 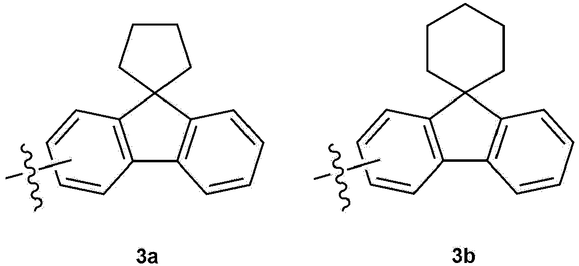
provided that when R_{b} is bonded to L₁, Ar₂ is the aryl.

2. The compound of claim 1,
wherein R₁, R₂, and R₄ is hydrogen.

3. The compound of claim 1,
wherein R₅ is hydrogen, or deuterium.

4. The compound of claim 1,
wherein L₁ is a single bond; a phenylene which is unsubstituted or substituted with one to four deuterium atoms; or a biphenylylene which is unsubstituted or substituted with one to eight deuterium atoms.

5. The compound of claim 1,
wherein L₂ is a single bond; a phenylene which is unsubstituted or substituted with one to four deuterium atoms; or a biphenylylene which is unsubstituted or substituted with one to eight deuterium atoms.

6. The compound of claim 1,
wherein R₃ is hydrogen.

7. The compound of claim 1,
wherein the compound represented by Chemical Formula 1 is represented by the following Chemical Formula 1-1 or 1-2: 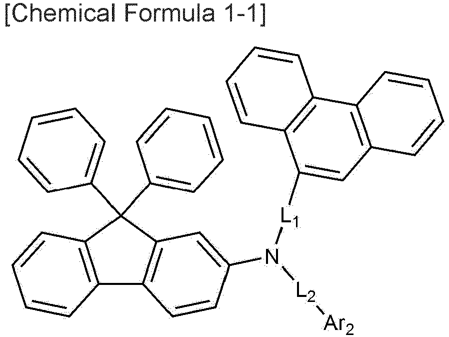 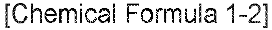 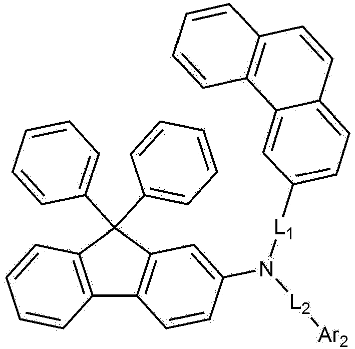 in Chemical Formulae 1-1 and 1-2,
L₁ and L₂ are each independently a single bond; a phenylene which is unsubstituted or substituted with one to four deuterium atoms; or a biphenylylene which is unsubstituted or substituted with one to eight deuterium atoms, and
Ar₂ is any one aryl group selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b; or any one heteroaryl selected from the group consisting of dibenzofuranyl and dibenzothiophenyl, wherein the Ar₂ is unsubstituted or substituted with one to five deuterium atoms:

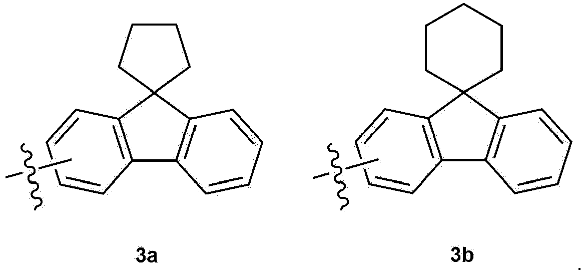

8. The compound of claim 1,
wherein the compound represented by Chemical Formula 1 is represented by the following Chemical Formula 1-3: 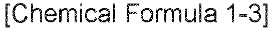 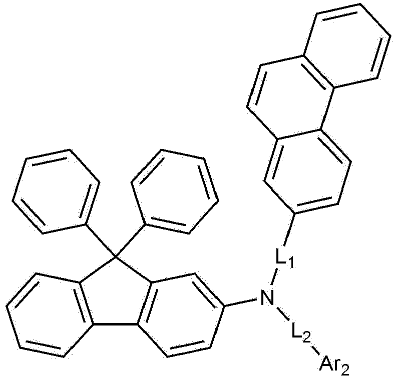 in Chemical Formula 1-3,
L₁ and L₂ are each independently a single bond; or a phenylene which is unsubstituted or substituted with one to four deuterium atoms; or a biphenylylene which is unsubstituted or substituted with one to eight deuterium atoms, and
Ar₂ is any one aryl group selected from the group consisting of phenyl, biphenylyl, terphenylyl, quaterphenylyl, naphthyl, dimethylfluorenyl, triphenylenyl, a substituent represented by the following Chemical Formula 3a, and a substituent represented by the following Chemical Formula 3b, wherein the Ar₂ is unsubstituted or substituted with one to five deuterium atoms:

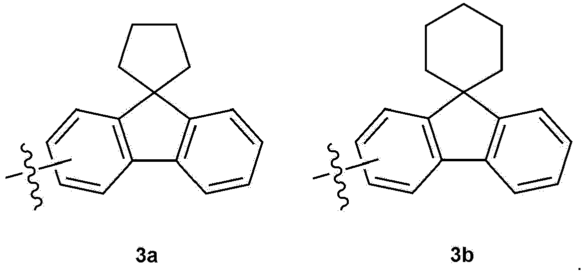

9. The compound of claim 1,
wherein the compound represented by Chemical Formula 1 is any one selected from the group consisting of the following:

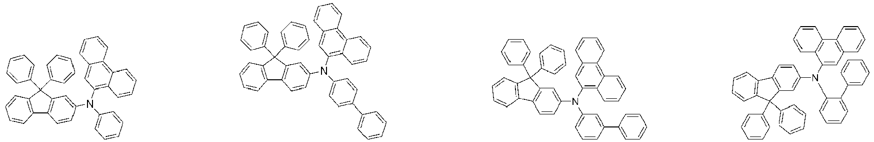

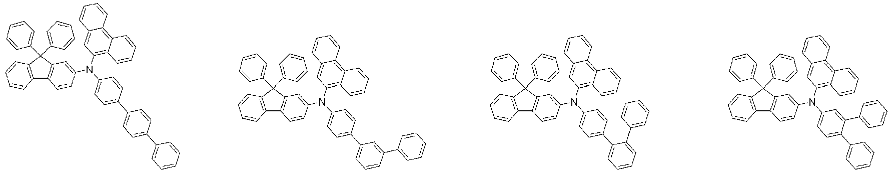

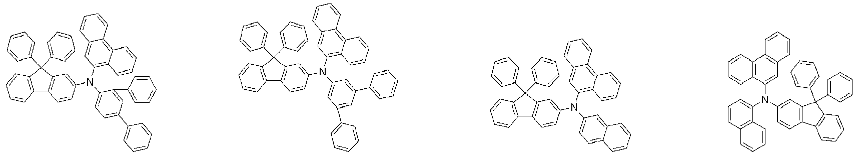

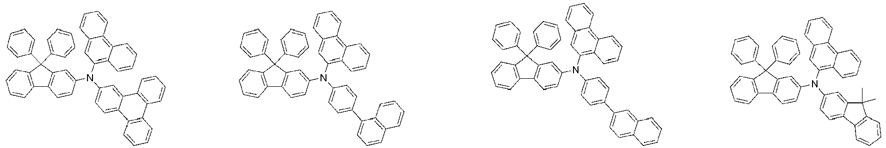

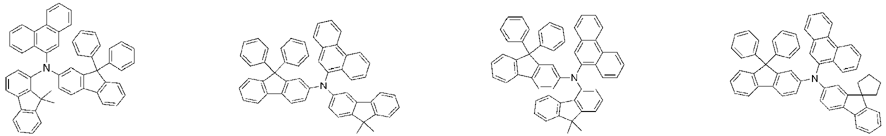

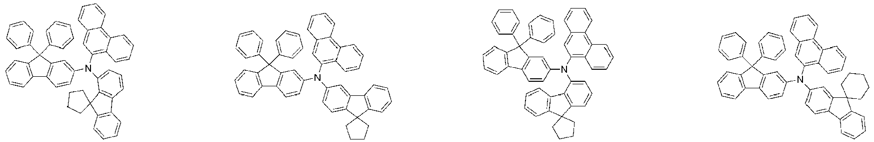

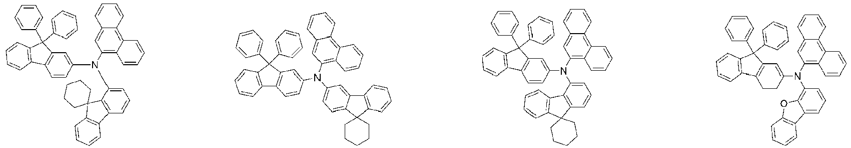

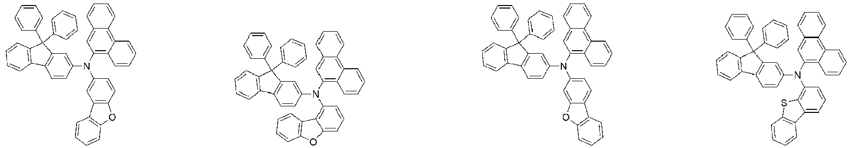

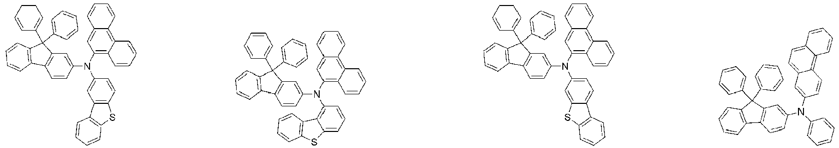

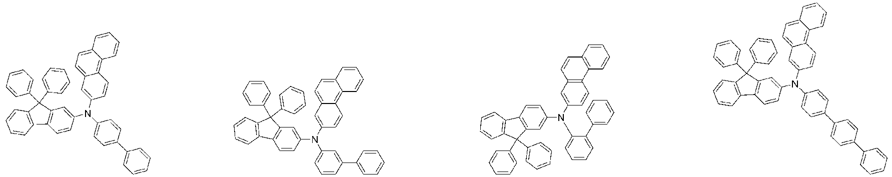

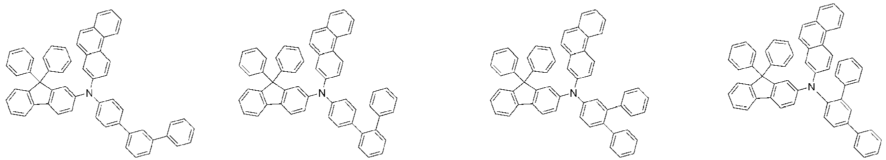

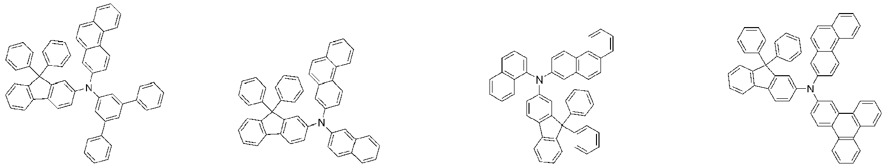

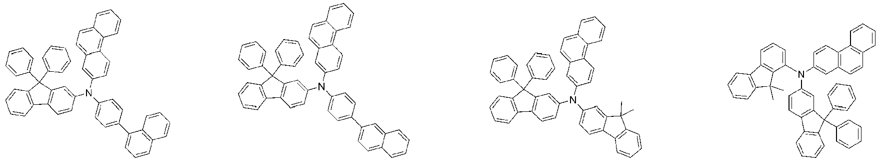

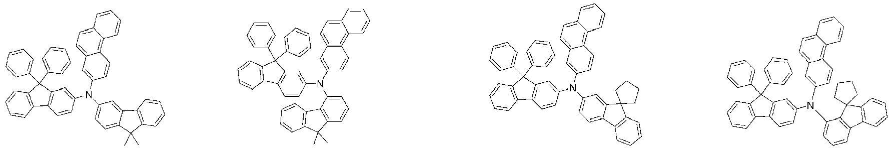

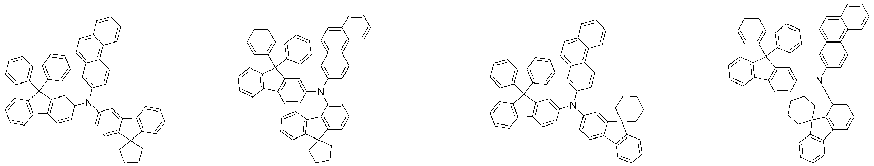

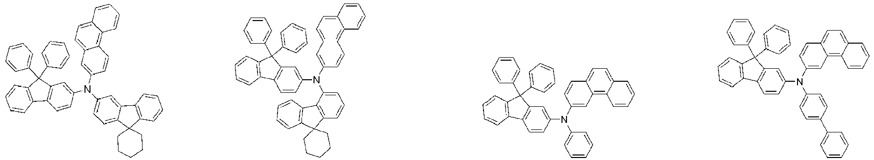

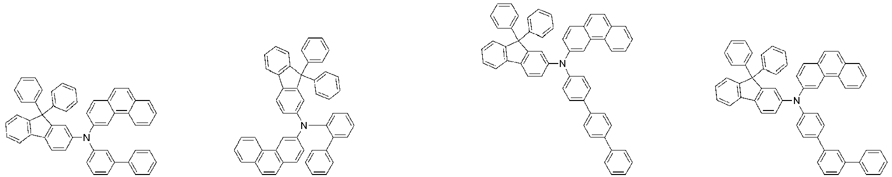

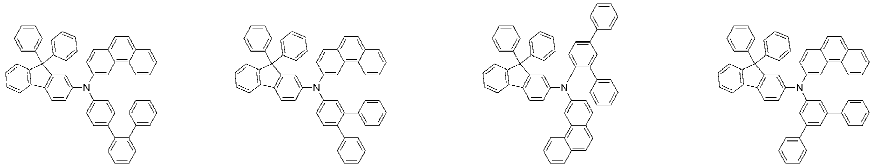

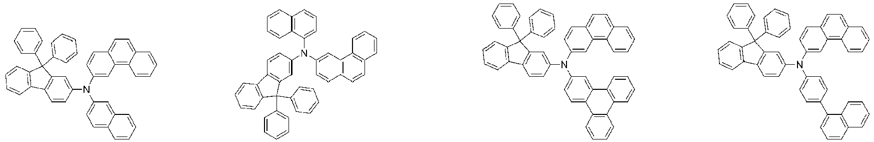

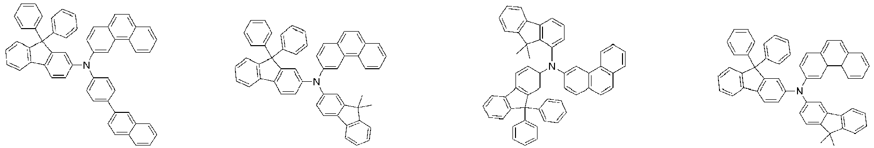

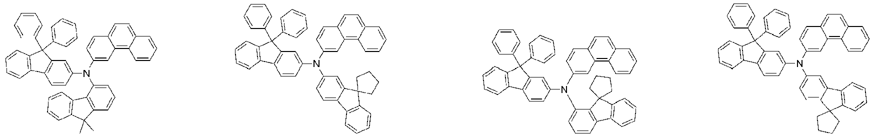

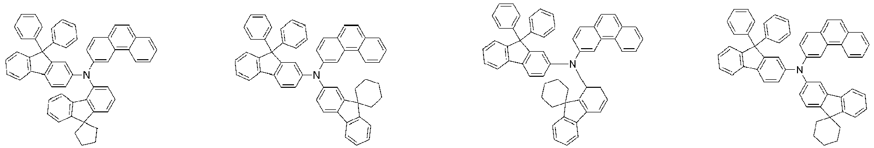

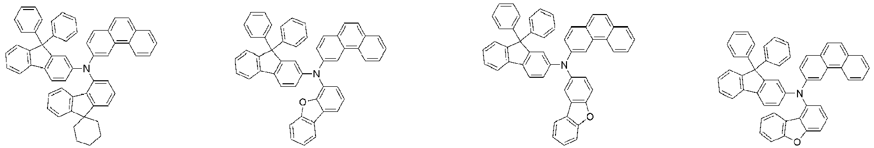

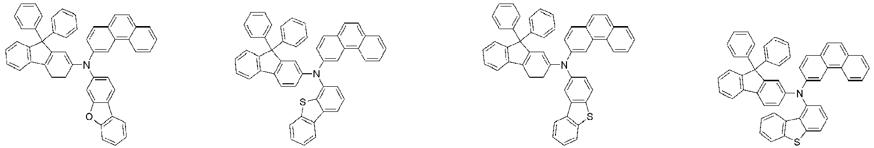

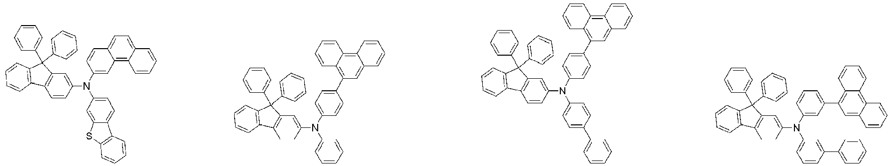

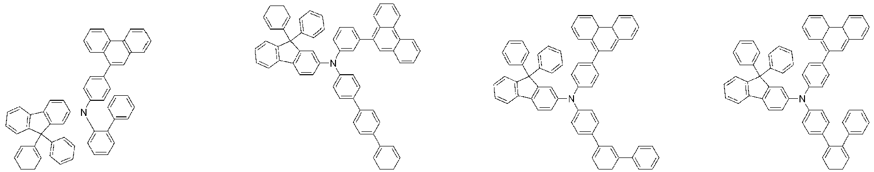

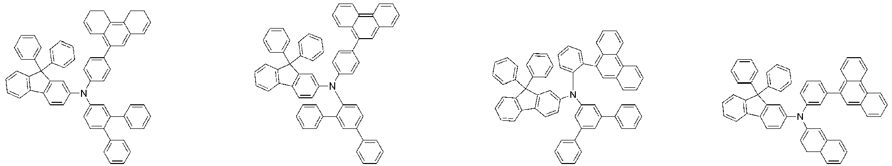

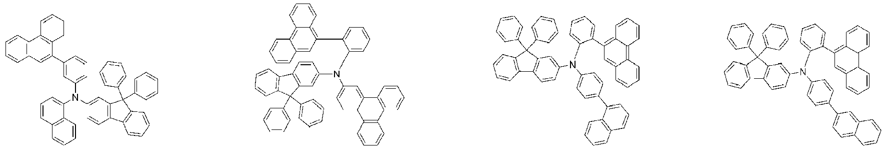

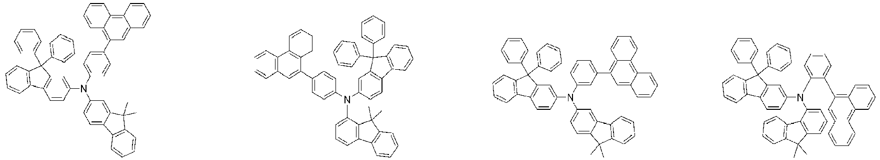

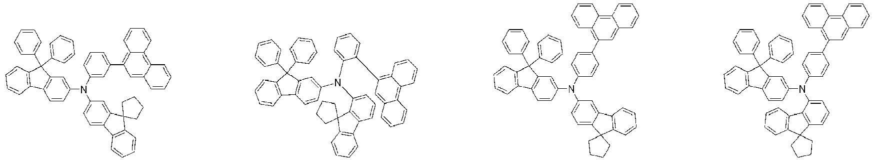

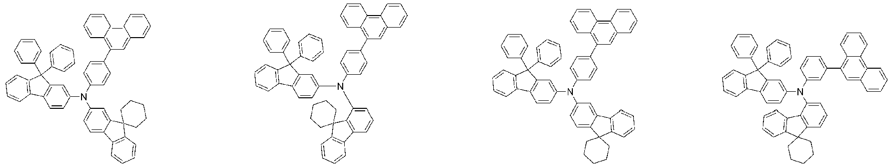

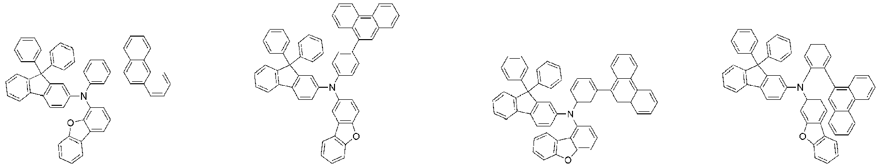

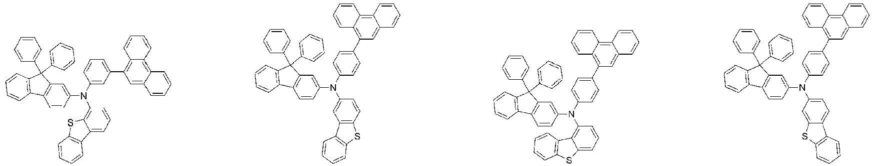

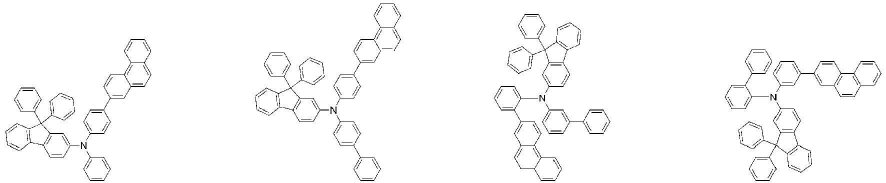

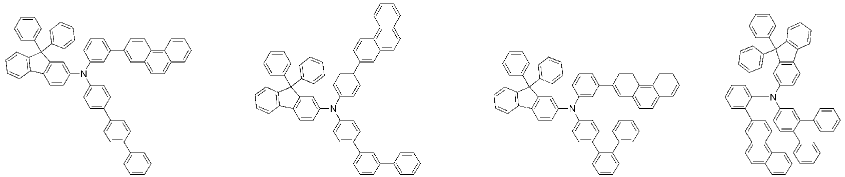

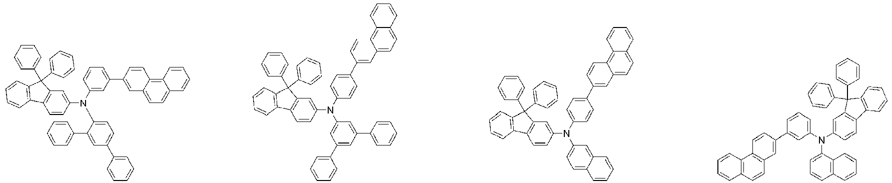

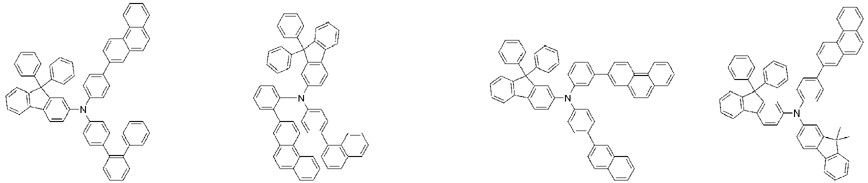

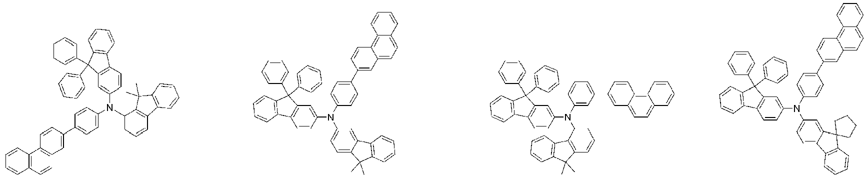

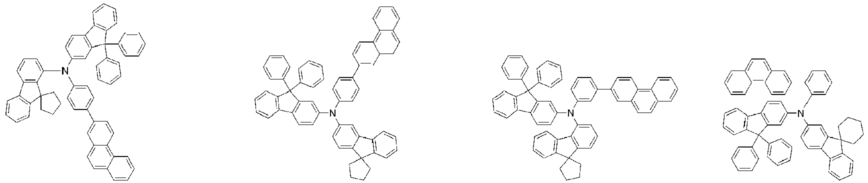

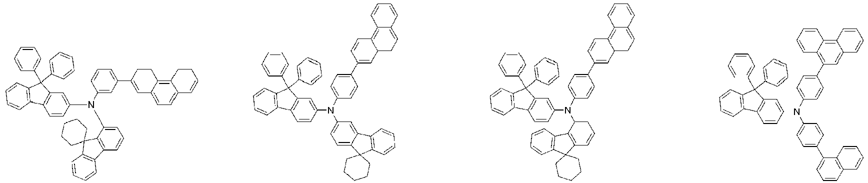

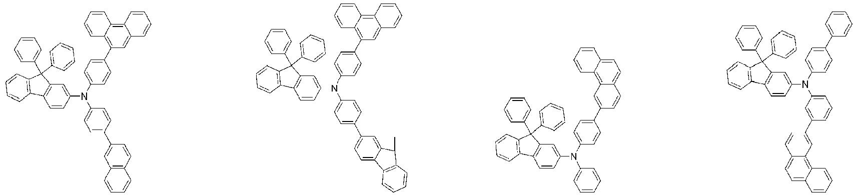

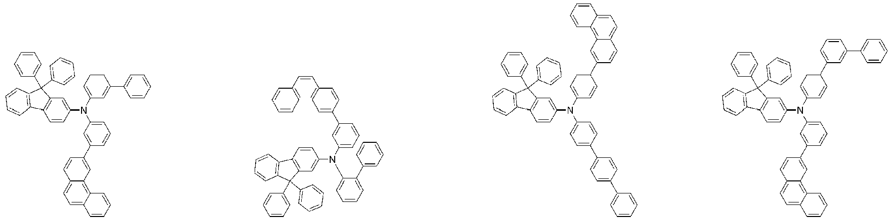

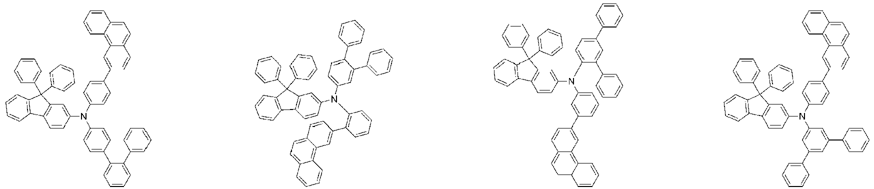

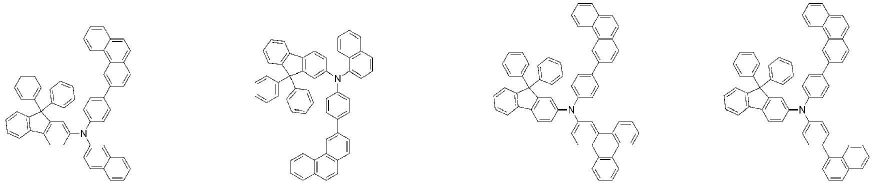

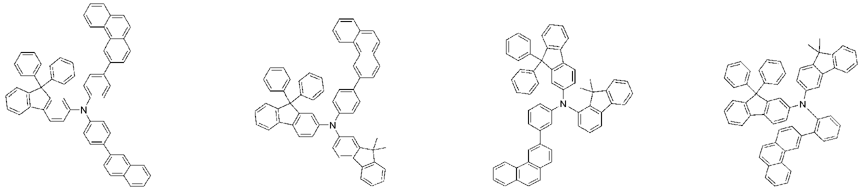

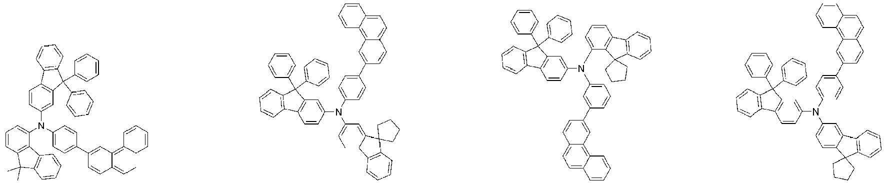

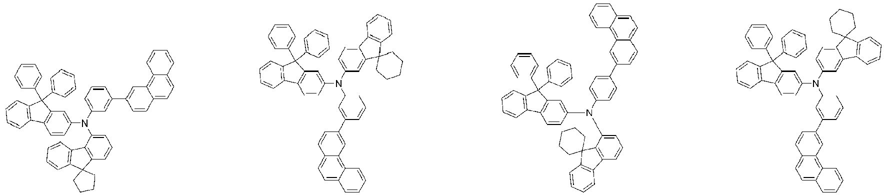

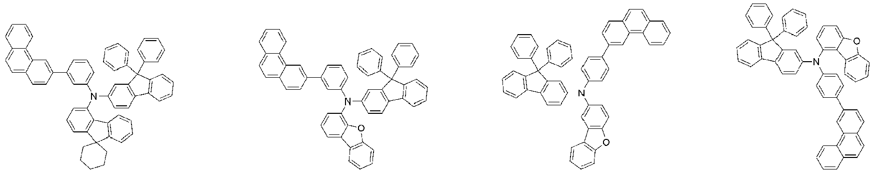

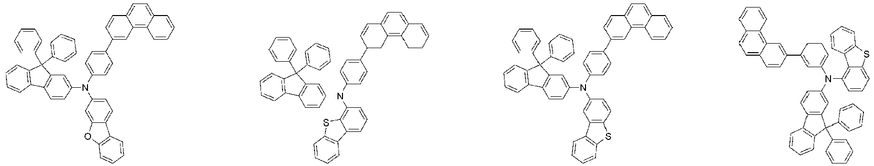

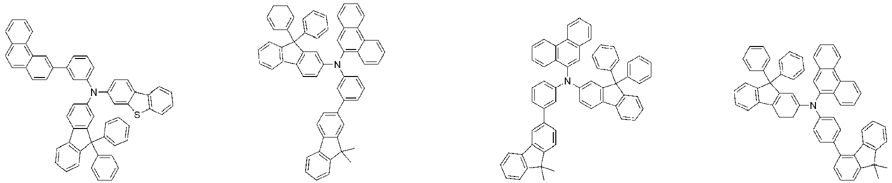

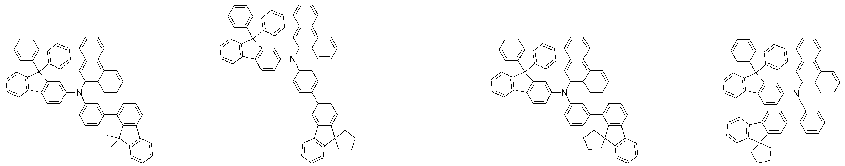

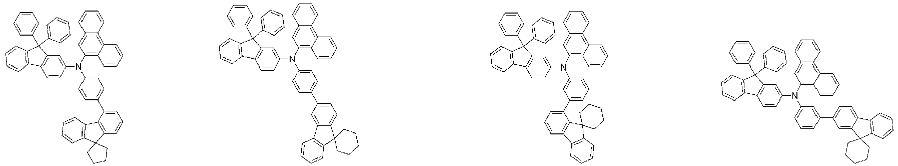

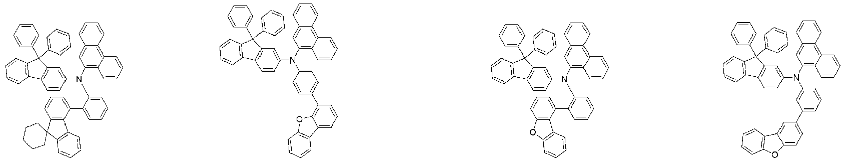

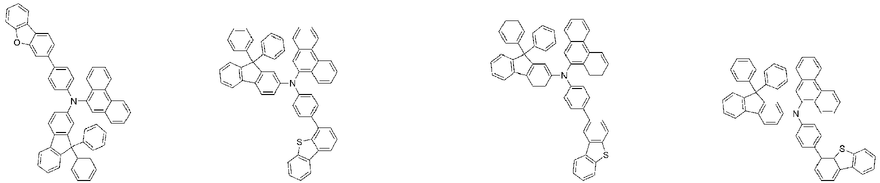

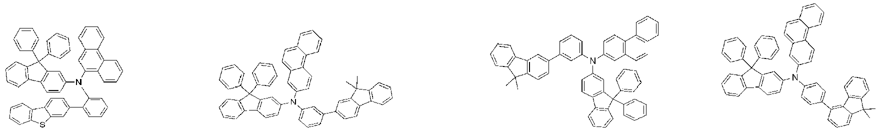

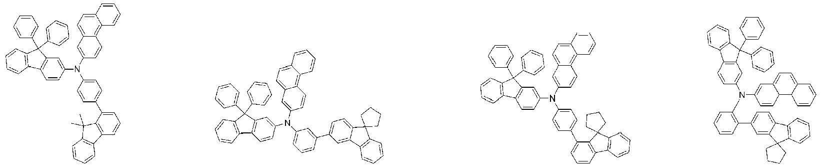

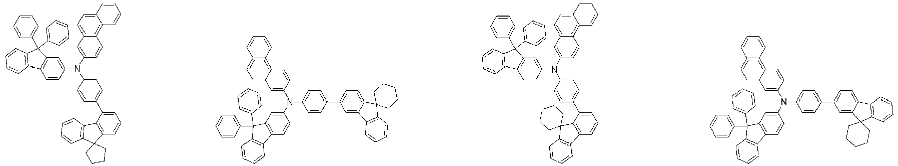

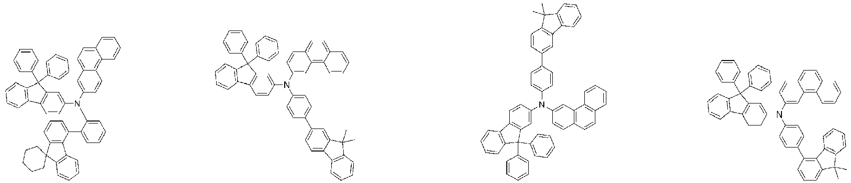

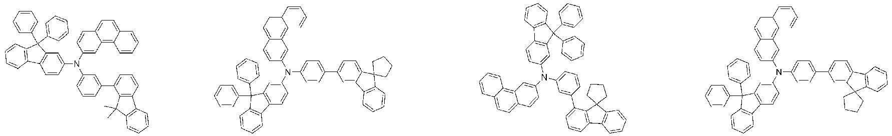

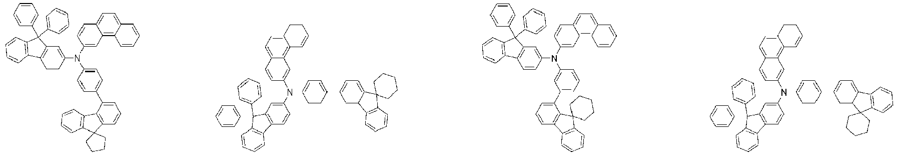

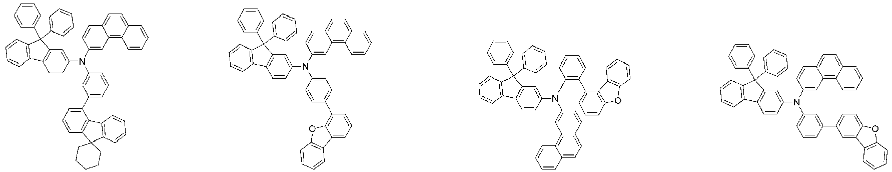

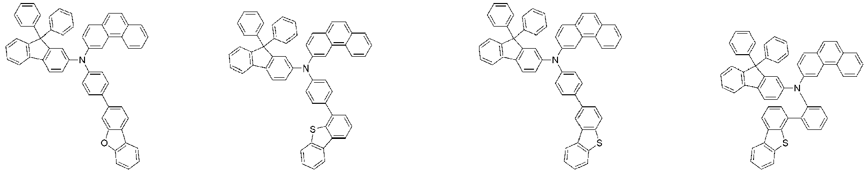

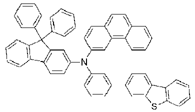

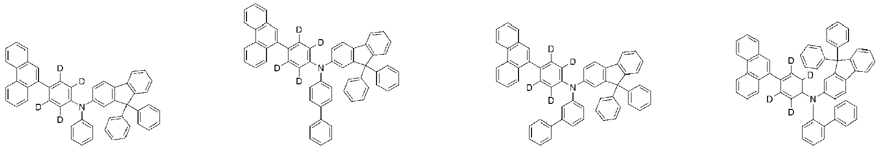

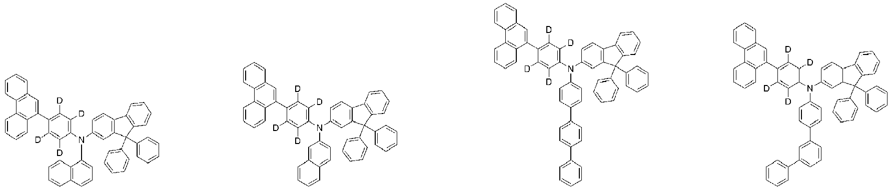

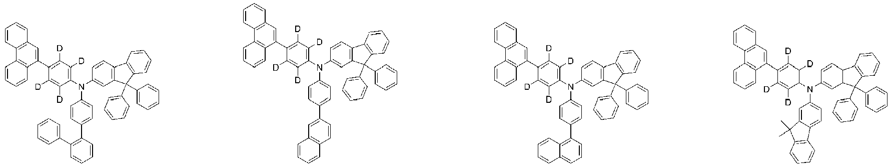

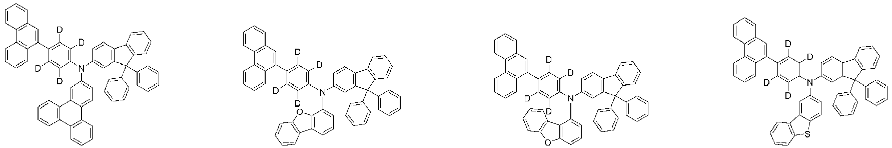

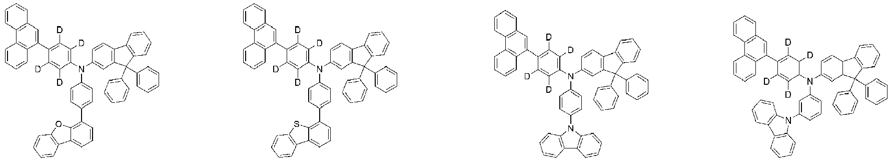

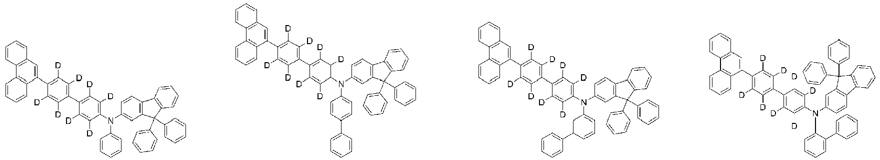

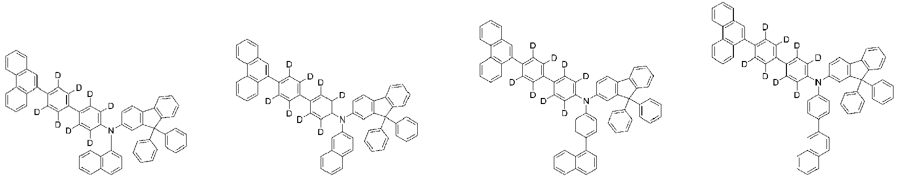

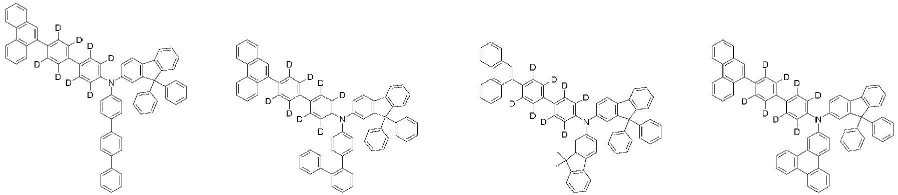

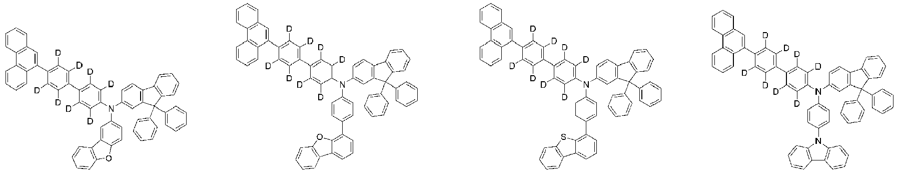

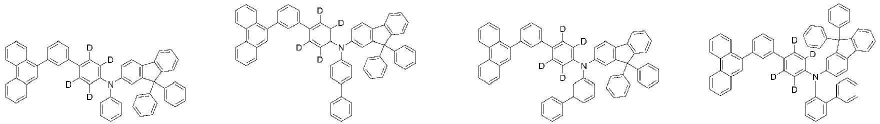

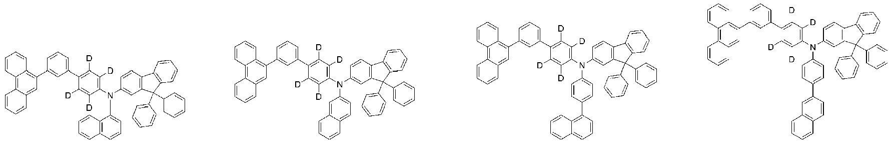

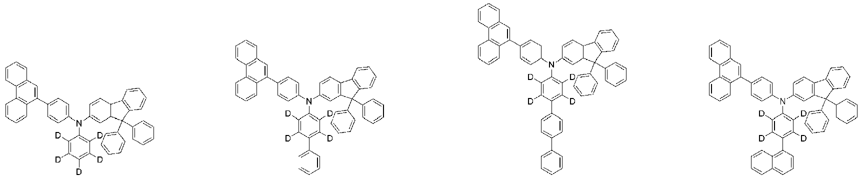

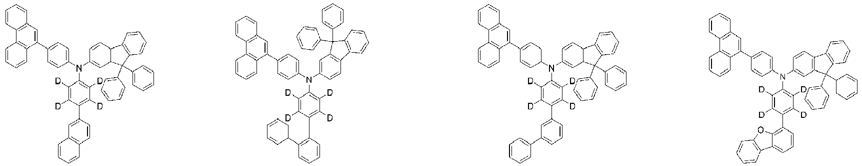

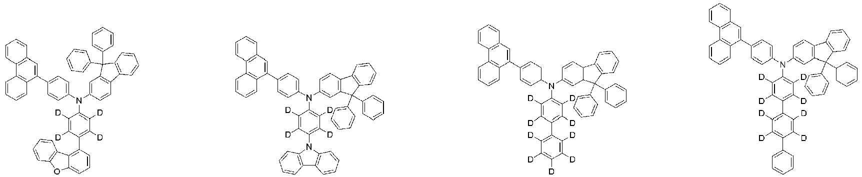

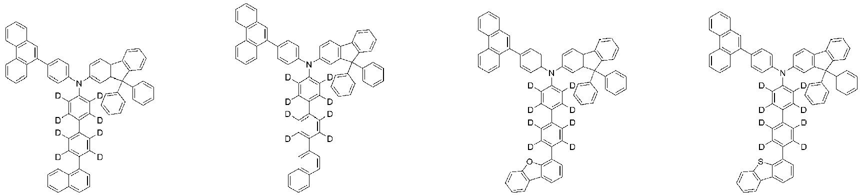

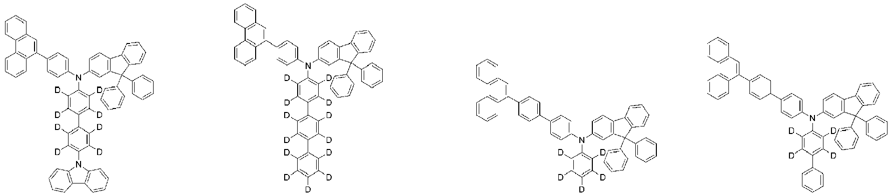

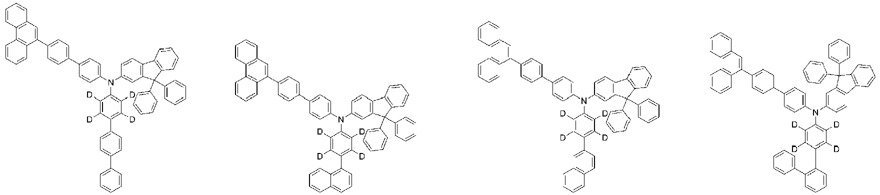

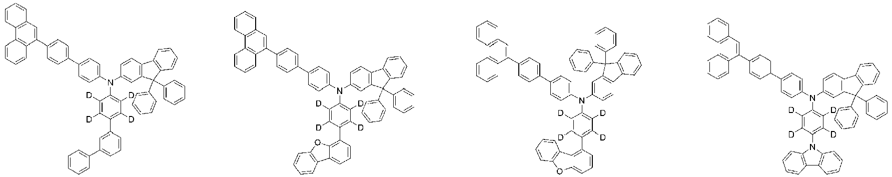

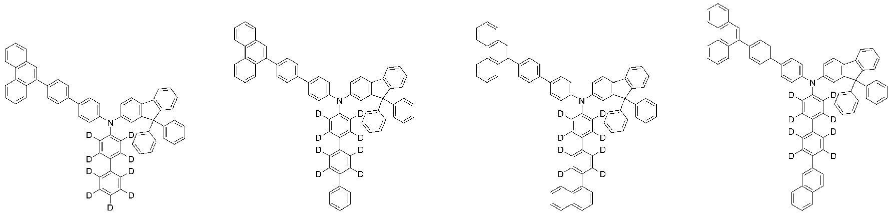

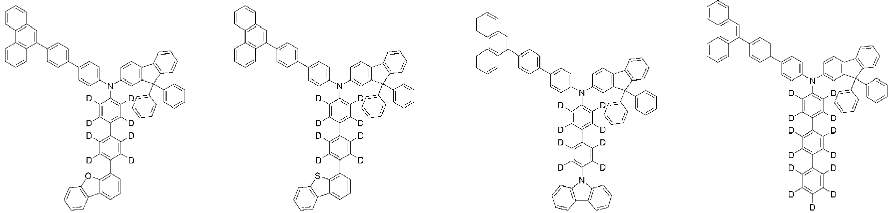

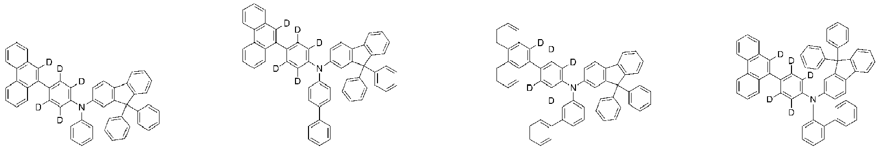

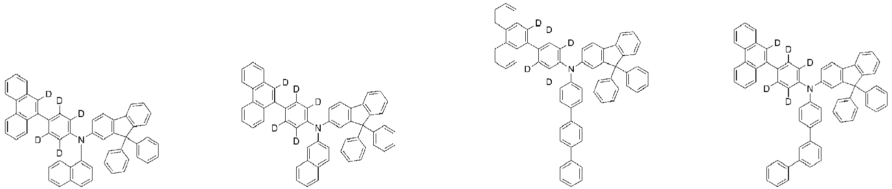

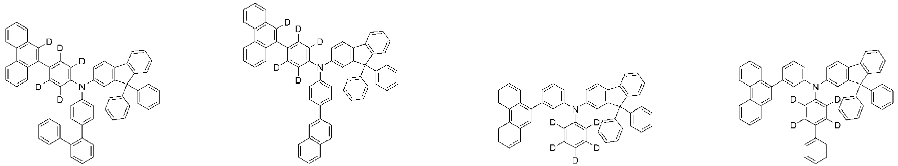

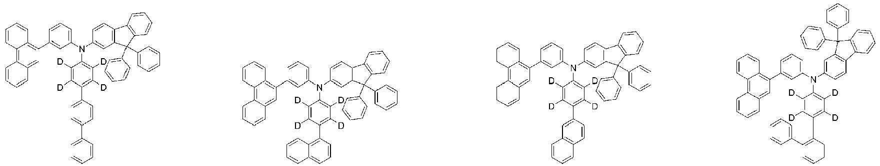

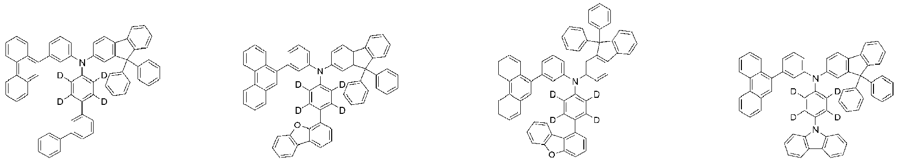

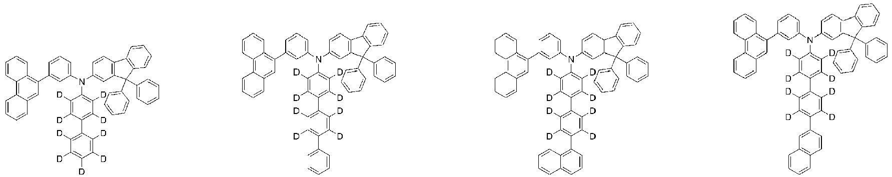

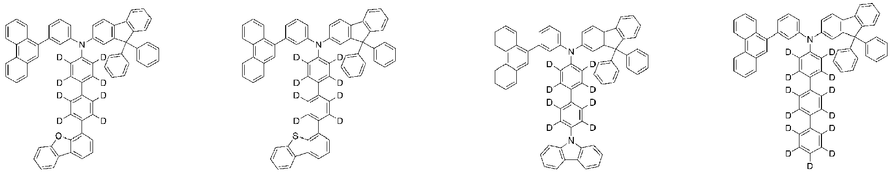

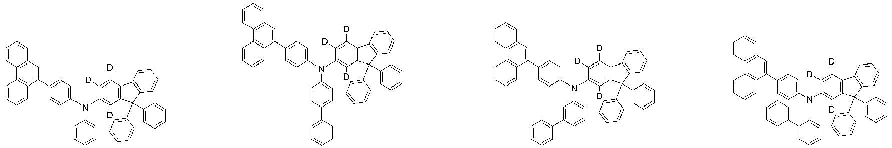

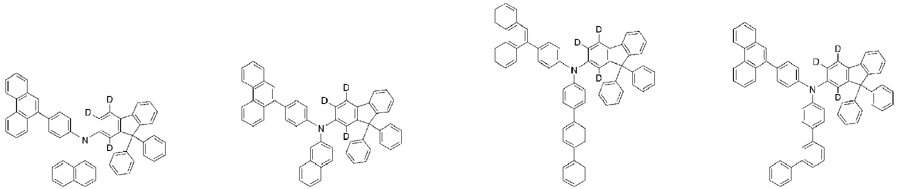

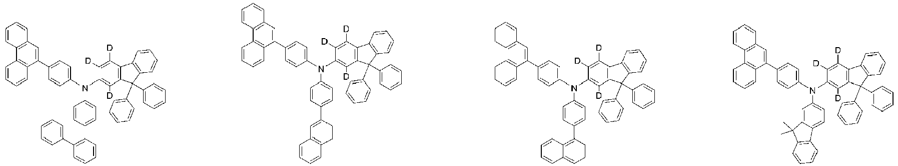

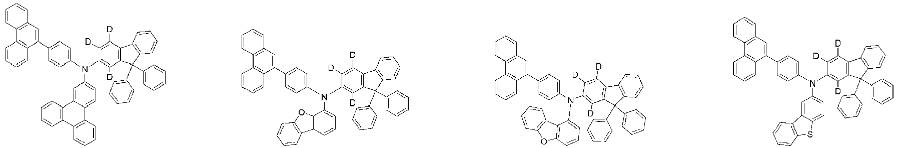

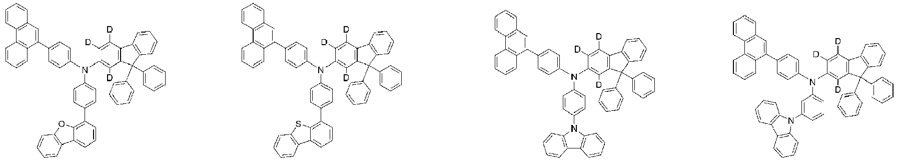

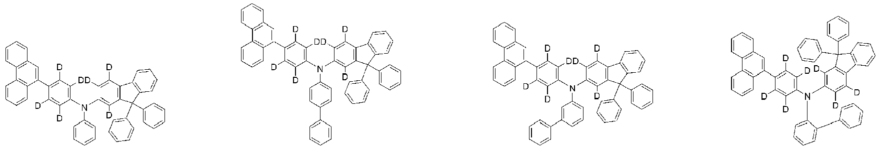

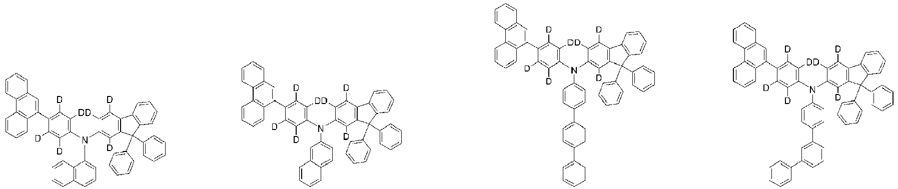

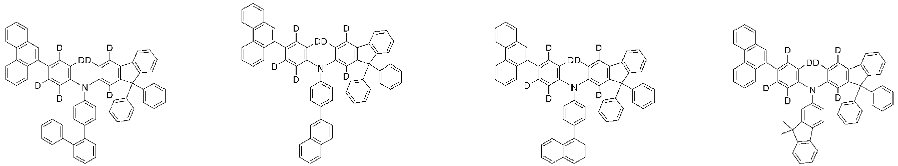

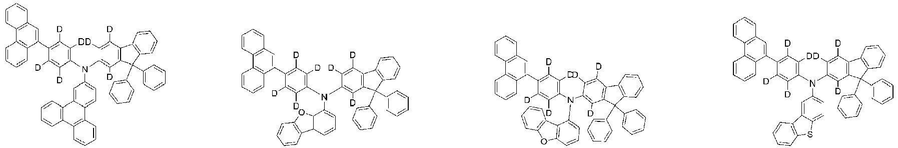

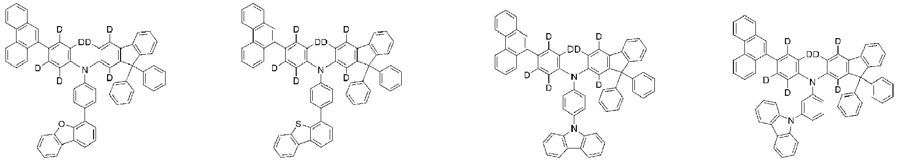

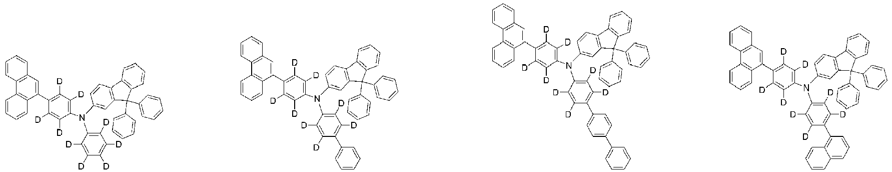

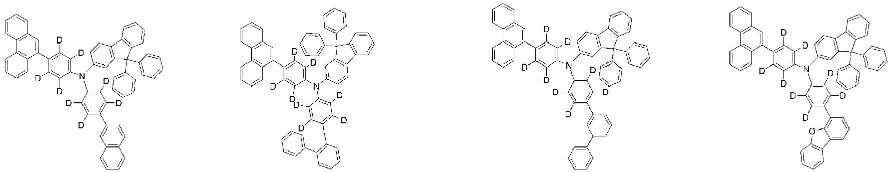

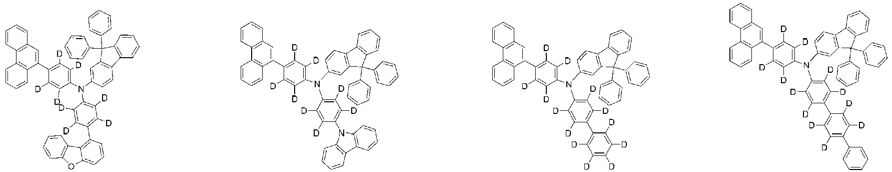

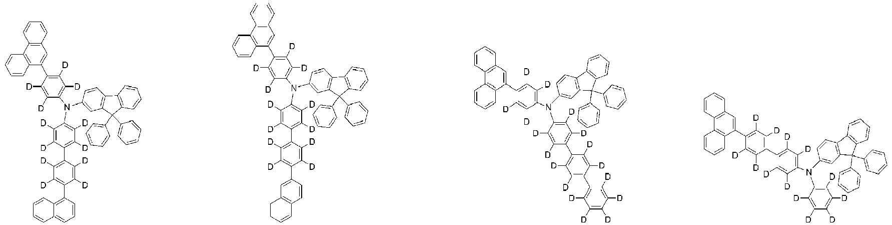

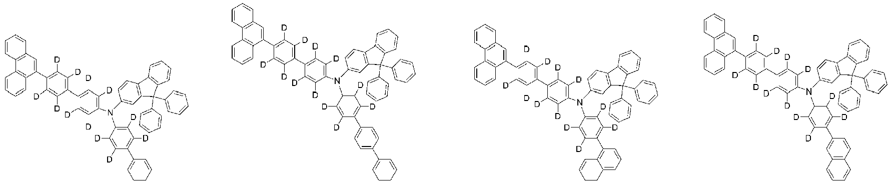

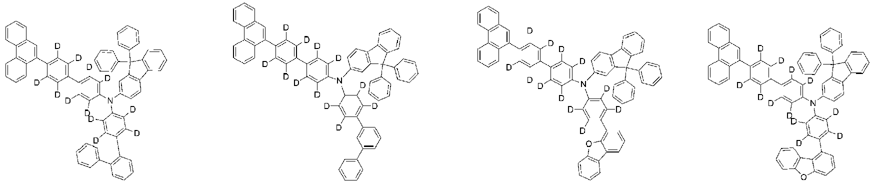

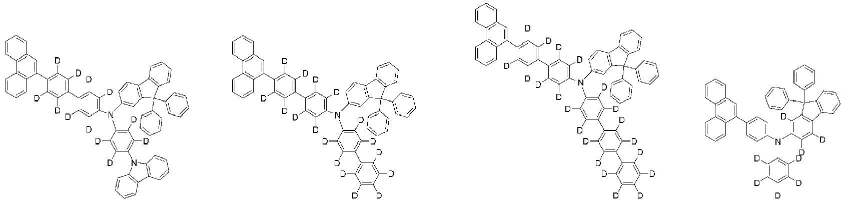

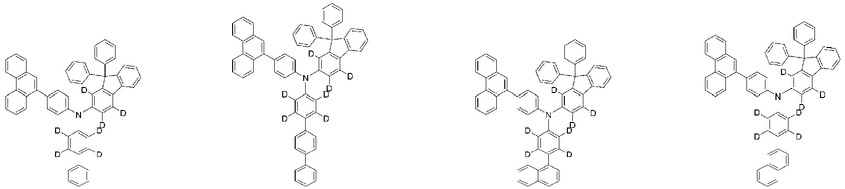

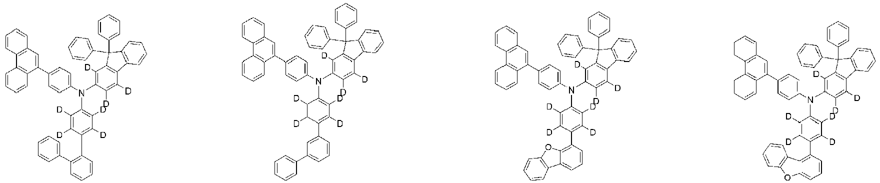

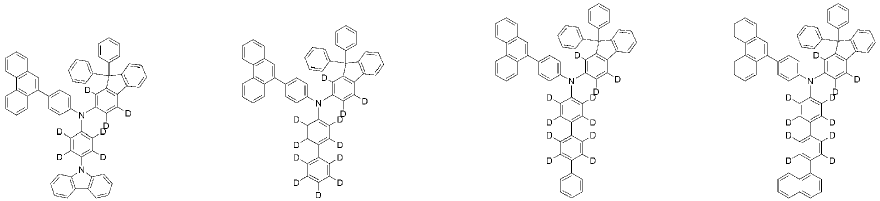

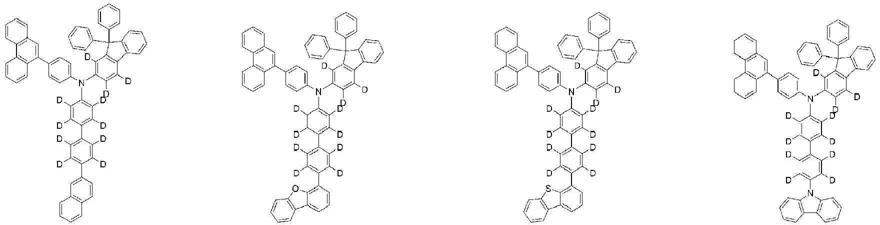

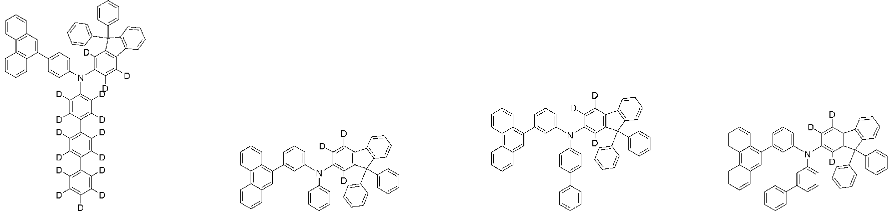

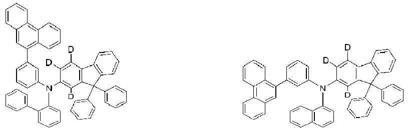

10. An organic light emitting device comprising a first electrode; a second electrode provided at a side opposite to the first electrode; and at least one layer of the organic material layers provided between the first electrode and the second electrode, wherein the at least one layer of the organic material layers includes a compound according to any one of claims 1 to 9.
